# FASCICULE DE BREVET EUROPEEN

(11) **EP 1 680 449 B1**
(45) Date de publication et mention de la délivrance du brevet: **14.10.2009**
(21) Numéro de dépôt: 04805265.8
(22) Date de dépôt: 21.10.2004
(51) Int. Cl.: C07K 16/46, A01K 67/027

(54) **MAMMIFERE NON-HUMAIN TRANSGENIQUE POUR LA REGION CONSTANTE DE LA CHAINE LOURDE DES IMMUNOGLOBULINES HUMAINES DE CLASSE A ET SES APPLICATIONS**
NICHT-HUMANER SÄUGER, WELCHER TRANSGEN IST FÜR DIE KONSTANTE REGION DER SCHWEREN KETTE VOM HUMANEM KLASSE A IMMUNOGLOBULIN UND VERWENDUNG DAFÜR
NON-HUMAN TRANSGENIC MAMMAL FOR THE CONSTANT REGION OF THE CLASS A HUMAN IMMUNOGLOBULIN HEAVY CHAIN AND APPLICATIONS THEROF

(30) Priorité: 24.10.2003 FR 0312502
(43) Date de publication de la demande: 19.07.2006
(73) Titulaire: CENTRE NATIONAL DE LA RECHERCHE SCIENTIFIQUE (CNRS), 75794 Paris Cedex 16 (FR); Universite de Limoges, 87032 Limoges Cedex 01 (FR)
(72) Inventeur: COGNE, Michel, F-87170 Isle (FR); SIRAC, Christophe, 19100 BRIVE (France) (FR); BARDEL, Micael, F-87270 Couzeix (FR); DECOURT, Catherine, Normale d'Instituteurs 87000 LIMOGES (FR); LE MORVAN, Caroline, F-87260 Vicq-Sur-Breuil (FR)
(74) Mandataire: Goulard, Sophie
(86) Numéro de dépôt international: PCT/FR2004/002701
(87) Numéro de publication internationale: WO 2005/047333

(56) Documents cités:
- WO-A-02/059154
- US-A- 5 545 807
- PINAUD, ERIC ET AL: "Localization of the 3' IgH locus elements that effect long-distance regulation of class switch recombination" IMMUNITY , 15(2), 187-199 CODEN: IUNIEH; ISSN: 1074-7613, 2001, XP011182522
- MAGADAN S ET AL: "Production of antigen-specific human monoclonal antibodies: comparison of mice carrying IgH/kappa or IgH/kappa/lambda transloci." BIOTECHNIQUES, 33 (3) 680, 682, 684 PASSIM. JOURNAL CODE: 8306785. ISSN: 0736-6205., septembre 2002 (2002-09), XP001182373
- CHAUVEAU C ET AL: "Insertion of the IgH locus 3' regulatory palindrome in expression vectors warrants sure and efficient expression in stable B cell transfectants" GENE, ELSEVIER BIOMEDICAL PRESS. AMSTERDAM, NL, vol. 222, no. 2, novembre 1998 (1998-11), pages 279-285, XP004150057 ISSN: 0378-1119
- RONAI D ET AL: "Use of a simple, general targeting vector for replacing the DNA of the heavy chain constant region in mouse hybridoma cells" JOURNAL OF IMMUNOLOGICAL METHODS, ELSEVIER SCIENCE PUBLISHERS B.V.,AMSTERDAM, NL, vol. 275, no. 1-2, 1 avril 2003 (2003-04-01), pages 191-202, XP004416768 ISSN: 0022-1759
- KHAMLICHI AHMED AMINE ET AL: "Immunoglobulin class-switch recombination in mice devoid of any Smu tandem repeat" BLOOD, vol. 103, no. 10, 15 mai 2004 (2004-05-15), pages 3828-3836, XP002319762 ISSN: 0006-4971

### SEQUENCE LISTING

<110> CENTRE NATIONAL DE LA RECHERCHE SCIENTIFIQUE
   COGNE, Michel
   SIRAC, Christophe
   BARDEL, Micael
   DECOURT, Catherine
   LE MORVAN, Caroline
<120> Mammifère non-humain transgénique pour la région constante de la chaîne lourde des immunoglobulines humaines de classe A et ses applications
<130> s644PCT115
<160> 6
<170> PatentIn version 3.1
<210> 1
   <211> 21
   <212> DNA
   <213> artificial séquence
<220>
   <223> amorce
   <400> 1
   gagtaccgtt gtctgggtca c 21
<210> 2
   <211> 23
   <212> DNA
   <213> artificial séquence
<220>
   <223> amorce
   <400> 2
   gagctctatg attattggtt aac 23
<210> 3
   <211> 22
   <212> DNA
   <213> artificial sequence
<220>
   <223> amorce
   <400> 3
   gcatgatctg gacgaagagc at 22
<210> 4
   <211> 22
   <212> DNA
   <213> artificial sequence
<220>
   <223> amorce
   <400> 4
   tcccctcaga agaactcgtc aa 22
<210> 5
   <211> 25
   <212> DNA
   <213> artificial sequence
<220>
   <223> amorce
   <400> 5
   aagtcgacat ggacatgagg gtgcc 25
<210> 6
   <211> 27
   <212> DNA
   <213> artificial sequence
<220>
   <223> amorce
<400> 6
   ttctcgagac ttaggtttaa tctccag 27

## Description

La présente invention est relative à un mammifère non-humain transgénique pour la région constante de la chaîne lourde des immunoglobulines humaines de classe A et à ses applications pour la production d'anticorps humanisés de classe IgA.

Les immunoglobulines A (IgA) comprennent deux chaînes lourdes identiques, d'isotype α1 (sous-classe IgA1) ou α2 (sous-classe IgA2) chez l'homme, associées par l'intermédiaire de ponts disulfures, à deux chaînes légères identiques d'isotype kappa (κ) ou lambda (λ).

La chaîne lourde α, qui est spécifique de cette classe d'immunoglobulines, existe sous forme membranaire et sous forme sécrétée. La forme sécrétée comprend quatre domaines d'environ 110 acides aminés : un domaine variable VH et trois domaines constants CH1, CH2 et CH3, ainsi qu'une région charnière (H) entre CH2 et CH3 et un octapeptide C-terminal. L'avant-dernière cystéine de cet octapeptide peut former une liaison covalente avec la chaîne J (ou pièce de jonction) qui sert à associer deux chaînes lourdes d'IgA de façon à former des IgA dimériques. La forme membranaire comprend en outre un domaine hydrophobe permettant l'ancrage de la protéine dans la membrane, ainsi qu'un domaine intracytoplasmique. La région de la chaîne lourde correspondant aux domaines CH1, CH2, H, CH3 associés, soit à l'octapeptide C-terminal (forme sécrétée), soit au domaine hydrophobe et intracytoplasmique (forme membranaire), est dénommée région constante par opposition à la région correspondant au domaine variable VH, qui est dénommée région variable.

Les chaînes légères κ et λ, qui sont communes à l'ensemble des classes et sous-classes d'immunoglobulines, comprennent deux domaines : un domaine variable (VL) et un domaine constant (CL). Chez l'Homme, l'expression des chaînes κ et λ est équivalente, alors que chez la souris l'expression du locus λ est très faible de telle sorte que 95 % des chaînes légères sont de type κ. La région de la chaîne légère correspondant au domaine CL est dénommée région constante par opposition à la région correspondant au domaine variable VL, qui est dénommée région variable.

Les gènes des immunoglobulines sont organisés en locus, un locus pour les chaînes lourdes (locus IgH) et un locus pour chacune des chaînes légères (locus lambda et kappa).

Les locus des chaînes légères comprennent chacun des gènes V et J codant pour le domaine variable et des gènes C codant pour le domaine constant ; au cours de la différenciation des lymphocytes B, un gène V est réarrangé avec un gène J et un gène C, et la région V subit en outre des mutations somatiques qui permettent de produire des anticorps de forte affinité pour l'antigène.

Le locus des chaînes lourdes comprend des gènes V, D et J codant pour le domaine variable et des gènes C (Cµ, Cδ, Cγ, Cε et Cα) codant pour les domaines constants des isotypes des différentes classes d'immunoglobulines ; chaque gène C, sauf Cδ, est précédé d'une séquence de commutation ou « switch « (S). Les gènes Cα (Cα1 et Cα2 chez l'homme) contiennent des introns séparant les exons codant pour les domaines constants CH1, CH2 et CH3 et l'exon de membrane (mb) ; la séquence codant pour la région charnière est incluse dans l'exon CH2. Au cours de la différenciation des lymphocytes B, un gène V est réarrangé avec un gène D et un gène J, et la région V subit également des mutations somatiques qui permettent de produire des anticorps de forte affinité pour l'antigène. En outre, alors que la réponse primaire à l'antigène est constituée principalement d'IgM, la réponse secondaire est associée au mécanisme de commutation de classe au cours duquel la séquence de commutation Sµ, située en amont de Cµ recombine avec une autre séquence de commutation conduisant ainsi la production d'une autre classe d'immunoglobuline (IgG, IgE ou IgA).

Le locus des chaînes lourdes d'immunoglobulines comprend également des séquences régulatrices en 5' (promoteur pVH et activateur intronique Eµ) et en 3' (activateurs de transcription Cα3'/hs3 reverse (hs3a), α3'E/hs1-2, hs3 (hs3b) et hs4). Il a été montré que la délétion des deux derniers activateurs en 3' (hs3b et hs4) réduisait sévèrement la transcription de la lignée germinale et la commutation de classe vers la plupart des isotypes d'immunoglobulines et pourrait également affecter l'expression du gène µ dans les cellules B au repos (Pinaud et al., Immunity, 2001, 15, 187-199). En outre, les séquences régulatrices 5' et 3' du locus des chaînes lourdes de la souris ont été utilisées pour construire un vecteur d'expression comprenant une cassette permettant une expression stable et efficace d'un transgène notamment un ADNc codant pour une chaîne légère d'immunoglobuline humaine dans des lignées de cellules B de souris ayant inséré cette cassette d'expression dans leur génome (Chauveau et al., Gene, 1998. 222, 279-285).

Le locus des chaînes lourdes d'immunoglobulines peut également être modifié par recombinaison homologue, notamment pour produire des immunoglobulines monoclonales de souris à partir d'hybridomes dont le locus IgH a été modifié à l'aide d'un vecteur de remplacement universel comprenant des régions d'homologies 5' et 3' constituées respectivement par le segment JH-Sµ et un fragment d'ADN en 3' du gène Cα (Ronai et al., Journal of Immunological Methods, 2003, 275, 191-202).

La diversité des anticorps produits en réponse à la stimulation par un antigène provient de la combinaison de plusieurs mécanismes : la multiplicité des gènes V, la mutation somatique de ces gènes V, la recombinaison somatique des gènes V et la recombinaison somatique des séquences de commutation.

Les IgA existent dans l'organisme sous deux formes différentes : une IgA sérique et une IgA sécrétoire (s-IgA).

L'IgA sérique représente 15 à 20 % des immunoglobulines sériques ; plus de 80 % de l'IgA sérique humaine est sous forme de monomère, alors que chez la plupart des autres espèces de mammifères, elle est essentiellement sous forme de dimère.

L'IgA secrétoire constitue la principale immunoglobuline des sécrétions (oculaires, salivaires, mammaires, trachéobronchiques et urogénitales), où elle existe sous la forme d'un dimère d'IgA associé à une autre protéine, le composant sécrétoire, qui est probablement enroulé autour du dimère d'IgA et attaché par des ponts disulfures au domaine CH2 de chaque monomère d'IgA. Contrairement à la chaîne J, la pièce sécrétoire n'est pas synthétisée par les plasmocytes mais par les cellules épithéliales. L'IgA dimérique sécrétée par les plasmocytes sous-épithéliaux se lie aux récepteurs poly-Ig présents au pôle basal des cellules épithéliales. Le complexe s-IgA/récepteur est alors endocyté et transporté à travers la cellule tout en restant fixé à la membrane des vésicules de transport. Celles-ci fusionnent avec la membrane plasmique à la surface luminale et libèrent l'IgA dimérique associée à la pièce sécrétoire qui provient du clivage du récepteur. Ainsi la pièce sécrétoire facilite le transport des IgA dans les sécrétions et les protège de la protéolyse.

Du fait de leur capacité à traverser l'épithélium des muqueuses et à prévenir l'entrée de pathogènes comme les virus, les bactéries, les parasites et les toxines, les IgA jouent un rôle majeur dans l'immunité locale : oculaire, respiratoire, digestive et urogénitale. Le mode d'action des IgA englobe des mécanismes actifs (activation du complément, liaison au récepteur Fc) et passifs (blocage des récepteurs des pathogènes (virus) et inhibition de la motilité des bactéries). Une corrélation étroite entre une réponse IgA spécifique et la protection contre une infection a été démontrée, notamment pour des virus (rotavirus, virus influenza, poliovirus, cyto-mégalovirus, virus respiratoire syncytial, virus Epstein-Barr). Des anticorps protecteurs de classe IgA dirigés contre de nombreux pathogènes humains (VIH, virus influenza A, bactéries, toxines , parasites) ont été isolés.

En raison de cette propriété particulière, les IgA ont des applications spécifiques pour le diagnostic et le traitement des maladies infectieuses et du cancer. Elles pourraient être utilisées en immunothérapie passive pour neutraliser des pathogènes (sérothérapie). Elles pourraient également être utilisées en immunothérapie active (vaccination) comme vecteur pour cibler des antigènes de tumeurs ou de micro-organismes pathogènes dans les muqueuses, de façon à induire une immunité locale spécifique de ces antigènes. En outre, elles sont utiles comme réactif fiable, sûr, stable et bien défini pour le diagnostic de maladies comme la maladie coeliaque, en remplacement des IgA humaines (IgA anti-transglutaminase, anti-endomysium, anti-gliadine) provenant des patients, qui exposent les manipulateurs à des risques de transmission de pathogènes humains (virus, prion).

Toutefois, le développement de ces applications est limité du fait qu'il n'existe pas de méthode efficace de production d'anticorps recombinants humains ou humanisés, de classe IgA.

On entend par anticorps humanisé, un anticorps dérivé d'un mammifère non-humain par fusion des domaines constants des chaînes lourdes et légères d'un anticorps humain avec les domaines variables des chaînes lourdes et légères d'un anticorps d'un mammifère non-humain.

En effet, les méthodes de productions d'anticorps recombinants humains ou humanisés actuellement disponibles présentent les inconvénients suivants :
* les méthodes *in vitro* reposent sur l'expression simultanée, à partir d'un ou plusieurs vecteurs recombinants, de chaînes lourdes et légères d'anticorps, d'une chaîne J, et éventuellement d'une pièce sécrétoire ; les chaînes lourdes et légères comprennent les domaines variables des chaînes lourdes et légères (VH et VL) d'un anticorps monoclonal humain ou murin d'intérêt, fusionnés respectivement aux domaines constants CH1, CH2 et CH3 d'une chaîne lourde α, et Cλ ou Cκ d'une chaîne légère humaine, ou bien les domaines VH et VL sont fusionnés à un domaine CH3 incluant l'octapeptide C-terminal (Demandes Internationales PCT WO 98/30577 et PCT WO 99/54484). Par exemple, la Demande Internationale PCT WO 98/30577 décrit la production *in vitro* à l'aide d'un ou plusieurs baculovirus recombinants, de mini-IgA dimériques (IgA-J) recombinantes humaines comprenant les domaines VH et VL d'un anticorps monoclonal murin ou humain, chacun fusionné à un domaine CH3 incluant l'octapeptide C-terminal, associés par l'intermédiaire d'une chaîne J ; une seule mini-IgA recombinante dirigée contre la gp120 du VIH, obtenue à partir d'un anticorps monoclonal humain neutralisant de classe IgG1 (anticorps S1-1), est décrite.
   Ces méthodes, qui sont spécifiques des IgA, sont limitées aux anticorps murins et aux quelques rares anticorps humains pour lesquels des hybridomes ont été isolés.
* les méthodes *in vivo* reposent sur la production d'immunoglobulines monoclonales humaines à partir de souris génétiquement modifiées possédant un transgène constitué par :
   - le locus IgH complet, le locus de la chaîne légère kappa et éventuellement le locus de la chaîne lambda humaine, dans leur configuration germinale, (Demande PCT WO 02/059154, Mendez et al., Nature Genetics, 1997, 15, 146-156 ; Green et Jakobovits, J. Exp. Med., 1998, 188, 483-495 et Demande de Brevet américain US n° 08/759,620 ; Magadan et al., Biotechniques, 2002, 33, 680-690),
   - un mini-locus IgH comprenant un ou plusieurs gènes VH, DH et JH, le gène Cµ et un second gène de la région constante, de préférence de la région Cγ. et le locus de la chaîne légère kappa (Demande PCT WO 02/059154, Brevet américain US 5,545,807), et
   - le locus IgH complet et le locus de la chaîne lambda dans sa configuration germinale (Demande de Brevet américain US n° 09/734,613). Lesdites souris sont éventuellement génétiquement invalidées pour le locus kappa endogène (souris κ -/-) et possèdent éventuellement une mutation qui inactive le locus IgH endogène (mutation µMT -/-).

   Ces méthodes ne permettent pas de produire de quantités importantes d'immunoglobulines humaines de classe IgA.

De manière surprenante, les Inventeurs ont construit des lignées de souris transgéniques qui produisent des quantités importantes d'immunoglobulines humanisées de classe IgA (de l'ordre du gramme par litre chez la souris). Les anticorps produits par ces animaux sont majoritairement des IgA humanisées ; ils ne contiennent pas d'IgM et seulement de très faibles quantités des autres classes d'immunoglobulines (IgG et IgE).

En conséquence, l'invention a pour objet un mammifère non-humain transgénique, **caractérisé en ce qu**'il comprend un locus IgH modifié par remplacement de la séquence de commutation Sµ par tout ou partie d'un transgène constitué par le gène Cα d'une immunoglobuline humaine de classe A, incluant au moins l'exon codant pour le domaine CH3 et l'exon de membrane.

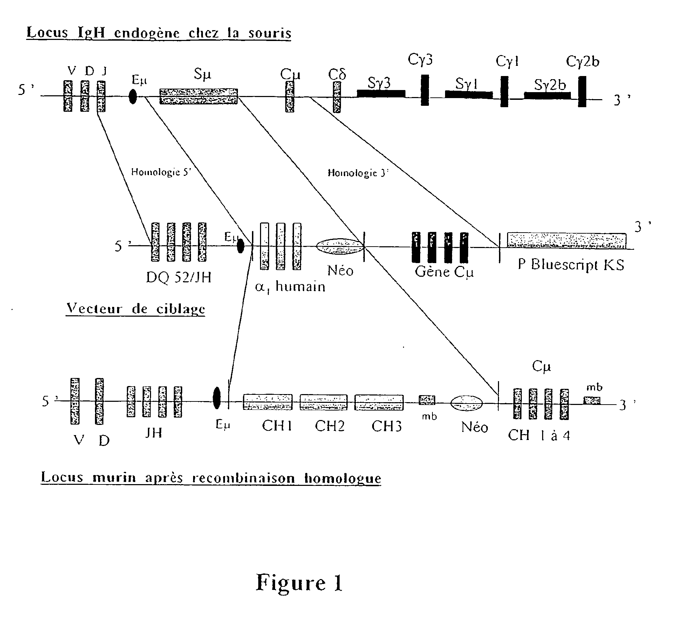

Conformément à l'invention, le transgène Cα ou la partie de ce transgène incluant au moins l'exon codant pour le domaine CH3 et l'exon de membrane, qui est inséré en lieu et place de la séquence de commutation Sµ, est donc situé entre l'activateur intronique Eµ, en 5' et le gène Cµ en 3'(figure 1).

Dans cette construction, la suppression de la séquence de commutation Sµ associée à l'insertion du transgène Cα en lieu et place de cette séquence, abolit l'expression du gène µ endogène responsable de la synthèse de chaînes lourdes d'IgM. En outre, celle des autres gènes de chaînes lourdes d'immunoglobulines est fortement diminuée du fait du blocage de la commutation de classe vers les gènes constants d'immunoglobulines situés en aval de Cµ sur le locus IgH endogène. Ainsi, les animaux transgéniques obtenus produisent des quantités importantes d'IgA chimériques dont le domaine constant de la chaîne lourde est humanisé et les domaines variables sont d'origine murine.

La chaîne lourde transgénique α humaine bénéficie d'un répertoire totalement diversifié puisque correspondant au répertoire normal généré par les réarrangements des segments VH, D et JH du locus IgH murin. En outre, les animaux transgéniques sont capables de produire des anticorps de forte affinité en réponse secondaire à l'antigène, du fait que leurs lymphocytes B peuvent recruter le phénomène d'hypermutation somatique.

Selon un mode de réalisation avantageux de l'invention, ledit mammifère non-humain transgénique est homozygote pour ledit locus IgH modifié.

Selon un autre un mode de réalisation avantageux de l'invention, ledit locus IgH est modifié par remplacement de la séquence de commutation Sµ par la totalité du gène Cα, incluant les exons CH1, CH2, CH3 et mb, séparés par les introns correspondants.

Selon un autre un mode de réalisation avantageux de l'invention, ledit locus IgH est modifié par remplacement de la séquence de commutation Sµ, par le segment du gène Cα incluant l'exon codant pour le domaine CH3 et l'exon de membrane.

Selon un autre un mode de réalisation avantageux de l'invention, ledit gène Cα est Cα1.

Selon encore un autre mode de réalisation avantageux de l'invention, ledit mammifère non-humain transgénique comprend un autre transgène codant pour une chaîne légère d'immunoglobuline humaine.

Selon une disposition avantageuse de ce mode de réalisation, la dite chaîne légère est une chaîne kappa.

De préférence, ledit transgène est un gène kappa humain comprenant l'activateur intronique Eµ, en amont et le palindrome *hs3a*/*hs1,2*/*hs3b*, en aval. Ces séquences, qui sont décrites dans Chauveau et al., Gene, 1998, 222, 279-285, permettent d'obtenir une forte expression de la chaîne kappa humaine dans les cellules B et d'induire l'hypermutation somatique du transgène kappa humain. De manière préférée, ledit transgène est sous le contrôle du promoteur de la chaîne lourde humaine (pVH).

Selon une autre disposition avantageuse de ce mode de réalisation, ledit mammifère non-humain transgénique est dizygote pour ledit transgène.

Selon une disposition avantageuse des modes de réalisation précédents de l'invention, lesdits mammifères non-humains transgéniques comprenant un autre transgène codant pour une chaîne légère kappa humaine possèdent un locus endogène de la chaîne légère kappa d'immunoglobulines inactivé (délété ou muté), notamment par recombinaison homologue. De préférence, lesdits mammifères non-humains transgéniques sont homozygotes pour ladite inactivation ; de manière préférée il s'agit de souris transgéniques. Parmi les mammifères non-humains transgéniques dont le locus endogène de la chaîne légère kappa d'immunoglobuline a été inactivé par recombinaison homologue, on peut citer notamment la lignée de souris décrite dans Zou et al., EMBO J., 1993, 12, 811-820.

De tels mammifères non-humains transgéniques produisent des IgA humanisés dont la quasi-totalité des chaîne légères sont d'origine humaine.

Selon une autre disposition avantageuse des modes de réalisation précédents de l'invention, lesdits mammifères non-humains transgéniques pour la chaîne lourde α1 et éventuellement pour la chaîne légère kappa humaine possèdent un locus endogène de la chaîne J inactivé (délété ou muté), notamment par recombinaison homologue. De préférence, lesdits mammifères non-humains transgéniques sont homozygotes pour ladite inactivation ; de manière préférée ils comprennent un autre transgène codant pour une chaîne J humaine, de manière encore plus préférée il s'agit de souris transgéniques. De tels mammifères non-humains transgéniques sont humanisés à la fois pour la production d'IgA et pour une protéine qui s'associe aux IgA, la chaîne J.

L'invention englobe les animaux transgéniques issus de n'importe quelle espèce de mammifère.

Selon un autre mode de réalisation avantageux de l'invention, ledit mammifère non-humain transgénique est une souris transgénique.

L'invention englobe notamment une lignée de souris double-transgénique, dénommée lignée HAMIGA pour « Humanized Antibodies Made Up Of Monoclonal Immunoglobulin A », comprenant :
- un locus IgH modifié par remplacement de la séquence de commutation Sµ par le gène Cα1 d'une immunoglobuline humaine de classe A, et
- un gène Vκ complet comprenant le gène VκI réarrangé avec un gène Jκ5, l'intron Jκ-Cκ et le gène Cκ, sous le contrôle transcriptionnel du promoteur de la chaîne lourde humaine (pVH), de l'activateur intronique Eµ en amont, et du palindrome *hs3a*/*hs1,2*/*hs3b* en aval.

Les animaux de cette lignée double-transgénique produisent des IgA partiellement humanisées pour la chaîne lourde et totalement humanisées pour ce qui concerne la chaîne légère.

En effet, l'expression de la chaîne kappa transgénique dans cette lignée est capable d'entraîner l'exclusion allélique, c'est-à-dire d'empêcher dans la plupart des cellules B transgéniques, l'expression des gène endogènes de chaînes légères d'immunoglobulines murines.

Le répertoire de réponse aux antigènes de cette lignée de souris est normal étant donné que c'est essentiellement le domaine VH de la chaîne lourde qui contribue à la formation du site anticorps. Or, la chaîne lourde transgénique α humaine bénéficie d'un répertoire totalement diversifié puisque correspondant au répertoire normal généré par les réarrangements des segments VH, D et JH du locus IgH murin, comme précisé ci-dessus.

En outre, les souris de cette lignée transgénique sont capables de produire des anticorps de forte affinité en réponse secondaire à l'antigène, du fait que leurs lymphocytes B peuvent recruter le phénomène d'hypermutation somatique, à la fois au niveau du gène de la chaîne lourde et du transgène de chaîne légère kappa.

Les animaux transgéniques selon l'invention sont obtenus par les procédés classiques de transgénèse animale, selon les protocoles standards tels que décrits dans Transgenic Mouse: Methods and Protocols ; Methods in Molecular Biology, Clifton, N.J., Volume. 209, Octobre 2002. Edité par : Marten H. Hofker, Jan Van Deursen, Martern H. Hofker et Jan Van Deursen. Publié par Holly T. Sklar : Humana Press*.*

Les séquences des gènes humains et murins d'immunoglobulines qui servent à la construction des animaux transgéniques selon l'invention sont connues et accessibles dans les bases de données. Par exemple, la séquence des exons CH1, CH2 et CH3 et de l'exon de membrane du gène Cα1 humain correspondent respectivement aux numéros d'accès J00220 et M60326 dans la base de données Genbank/EMBL.

La construction du gène Vκ est telle que décrite dans Chauveau et al., Gene, 1998, 222, 279-285 ; la séquence du gène VκI réarrangé avec le gène Jκ5 et le gène Cκ correspond à la séquence présentant le numéro d'accès X64133 dans la base de données EMBL/Genbank, qui code pour une chaîne légère humaine présentant la séquence correspondant au numéro d'accès CAA45494 dans la base de données EMBL.

Les insertions de fragments géniques dans le génome des mammifères non-humains peuvent être réalisées de façon aléatoire, de préférence elles sont réalisées de façon ciblée, par recombinaison homologue avec un vecteur de ciblage approprié comprenant éventuellement des séquences de recombinaison d'une recombinase site-spécifique comme les sites LoxP de la recombinase Cre. Les inactivations ou délétions de fragments géniques dans le génome des mammifères non-humains sont réalisées par recombinaison homologue avec un vecteur de ciblage approprié comprenant éventuellement des séquences de recombinaison d'une recombinase site-spécifique comme les sites LoxP de la recombinase. Les animaux double-transgéniques sont obtenus par croisement d'animaux transgéniques pour la chaîne lourde alpha avec des animaux transgéniques pour la chaîne légère, tels que définis ci-dessus. Les animaux double-transgéniques sont éventuellement croisés avec des animaux transgéniques dont le locus endogène de la chaîne légère kappa d'immunoglobulines a été inactivé par recombinaison homologue et/ou avec des animaux dont le locus endogène de la chaîne J d'immunoglobulines a été inactivé et qui possèdent de surcroit un transgène J humain, tels que définis ci-dessus.

La présente invention a également pour objet un vecteur de ciblage de recombinaison homologue, **caractérisé en ce qu**'il comprend le gène Cα d'une immunoglobuline humaine de classe A ou un segment de ce gène incluant au moins l'exon codant pour le domaine CH3 et l'exon de membrane, flanqué des fragments de séquences du locus IgH d'un mammifère non-humain qui sont adjacents à la séquence Sµ.

Selon un mode de réalisation avantageux dudit vecteur de ciblage, il comprend une cassette d'expression d'un marqueur de sélection approprié, adjacente audit gène Cα ou au segment dudit gène tel que défini ci-dessus.

Selon une disposition avantageuse de ce mode de réalisation, ladite cassette d'expression est flanquée de séquences de recombinaison site-spécifique. De préférence, lesdites séquences sont les séquences LoxP de la recombinase Cre. Cette disposition permet éventuellement d'exciser ladite cassette d'expression.

Selon un autre mode de réalisation dudit vecteur de ciblage, lesdits fragments de séquences qui sont adjacents à la séquence Sµ sont d'origine murine.

Selon un autre mode de réalisation dudit vecteur de ciblage, le gène Cα ou le segment dudit gène est flanqué en 5' d'un fragment d'environ 5 kb correspondant à la région JH/Eµ et en 3' d'un fragment d'environ 5 kb correspondant à la région Cµ, lesquels fragments correspondant respectivement aux positions 131281 à 136441 et 140101 à 145032 dans la séquence du chromosome 12 murin (numéro d'accès AC073553 dans la base de données EMBL/Genbank).

La présente invention a également pour objet des cellules embryonnaires d'un mammifère non-humain, modifiées par un vecteur de ciblage tel que défini ci-dessus.

Les dites cellules embryonnaires (cellules souches totipotentes) modifiées sont utiles pour l'obtention des mammifères transgéniques tels que définis ci-dessus ; elles sont injectées dans des blastocystes de mammifère, selon les techniques classiques de transgénèse animale.

La présente invention a également pour objet, l'utilisation d'un mammifère non-humain transgénique tel que défini ci-dessus pour la production d'anticorps humanisés de classe IgA ou de fragments de ces anticorps.

La présente invention a également pour objet un procédé de préparation d'anticorps humanisés de classe IgA ou de fragments de ces anticorps, **caractérisé en ce qu**'il comprend au moins les étapes suivantes :
- l'immunisation d'un mammifère non-humain transgénique tel que défini ci-dessus avec un antigène d'intérêt,
- l'obtention par tout moyen approprié, d'anticorps humanisés de classe IgA ou de fragments de ces anticorps, à partir du sérum, des sécrétions ou des lymphocytes B dudit mammifère non-humain transgénique préalablement sacrifié.

Les mammifères non-humains transgéniques selon l'invention présentent l'avantage de permettre la production d'anticorps monoclonaux de classe IgA qui sont d'emblée des anticorps chimériques humanisés de classe IgA. Le procédé de production d'anticorps monoclonaux humanisés de classe IgA selon l'invention est donc plus simple, plus rapide et plus économique que les procédés de l'art antérieur puisqu'il ne nécessite pas d'étapes additionnelles de clonage des gènes desdits anticorps et de fusion des domaines variables desdits anticorps avec les domaines constants d'immunoglobulines humaines.

L'invention englobe la production d'anticorps polyclonaux ou monoclonaux constitués par des IgA monomériques, dimériques et des s-IgA, ainsi que de leurs fragments, notamment les fragments Fab, Fab'2 et Fc.

Les anticorps humanisés de classe IgA tels que définis ci-dessus et leurs fragments sont préparés par les techniques classiques connues de l'Homme du métier, telles que celles décrites dans Antibodies : A Laboratory Manual, E. Howell and D Lane, Cold Spring Harbor Laboratory, 1988*.*

De manière plus précise :
- les anticorps polyclonaux sont préparés par immunisation d'un mammifère non humain transgénique tel que défini ci-dessus avec un antigène d'intérêt, éventuellement couplé à la KLH ou à l'albumine et/ou associé à un adjuvant approprié tel que l'adjuvant de Freund (complet ou incomplet) ou l'hydroxyde d'alumine ; après obtention d'un titre en anticorps satisfaisant, les anticorps sont récoltés par prélèvement du sérum des animaux immunisés et enrichis en IgA par précipitation, selon les techniques classiques, puis les IgA spécifiques sont éventuellement purifiées par chromatographie d'affinité sur une colonne appropriée sur laquelle est fixé l'antigène tel que défini ci-dessus, de façon à obtenir une préparation d'IgA monospécifiques.
- les anticorps monoclonaux sont produits à partir d'hybridomes obtenus par fusion de lymphocytes B d'un mammifère non-humain transgénique tel que défini ci-dessus avec des myélomes, selon la technique de Kôhler et Milstein (Nature, 1975, 256, 495-497) ; les hybridomes sont cultivés *in vitro*, notamment dans des fermenteurs ou produits *in vivo*, sous forme d'ascite ; alternativement, lesdits anticorps monoclonaux sont produits par génie génétique comme décrit dans le brevet américain US 4,816,567. Par exemple, des mammifères non-humains transgéniques tels que défini ci-dessus sont immunisés de façon forte et répétée avec des antigènes choisis (antigènes de bactéries, de virus, de champignons, antigènes spécifiques de tumeurs tels que l'antigène carcino-embryonnaire, etc...), selon un protocole standard comprenant une première immunisation par injection intrapéritonéale de l'antigène dans un volume équivalent d'adjuvant complet de Freund puis une deuxième immunisation (rappel) 15 jours plus tard dans des conditions identiques mais avec cette fois de l'adjuvant incomplet de Freund. Les anticorps monoclonaux sont produits selon un protocole standard comprenant le sacrifice des animaux deux semaines après le dernier rappel, le prélèvement de la rate, la mise en suspension des lymphocytes spléniques et la fusion de ces lymphocytes avec la lignée cellulaire SP2/0 (cette lignée murine ne produit aucun anticorps murin, est immortalisée, et possède toute la machinerie de sécrétion nécessaire à la sécrétion d'immunoglobulines).
- les fragments d'anticorps sont produits à partir des régions V_{H} et V_{L} clonées, à partir des ARNm d'hybridomes ou de lymphocytes spléniques d'un mammifère non-humain transgénique selon l'invention, immunisé ; par exemple, les fragments Fv ou Fab sont exprimés à la surface de phages filamenteux selon la technique de Winter et Milstein (Nature, 1991, 349, 293-299) ; après plusieurs étapes de sélection, les fragments d'anticorps spécifiques de l'antigène sont isolés et exprimés dans un système d'expression approprié, par les techniques classiques de clonage et d'expression d'ADN recombinant.

Les anticorps ou leur fragments tels que définis ci-dessus, sont purifiés par les techniques classiques connues de l'Homme du métier, telles que la chromatographie d'affinité.

La présente invention a également pour objet, un anticorps humanisé de classe IgA susceptible d'être obtenu par le procédé tel que défini ci-dessus, **caractérisé en ce qu**'il comprend une chaîne lourde chimérique dont le(s) domaine(s) constant(s) sont d'origine humaine et une chaîne légère humaine dont le domaine variable est codé par VκI-Jκ5.

L'invention englobe les anticorps humanisés de classe IgA dont la chaîne légère est codée par le gène VκI-Jκ5 présentant la séquence EMBL/Genbank X64133 ou une séquence produite par hypermutation de cette séquence, notamment après activation des lymphocytes B en présence de l'antigène.

La présente invention a également pour objet, un fragment d'un anticorps humanisé de classe IgA susceptible d'être obtenu par le procédé tel que défini ci-dessus, **caractérisé en ce qu**'il comprend un fragment desdites chaînes lourdes et légères telles que définies ci-dessus.

L'invention englobe les anticorps polyclonaux, les anticorps monoclonaux ainsi que leurs fragments (Fab, Fc, Fab'2).

Les anticorps humanisés selon l'invention et leurs fragments tels que définis ci-dessus, sont bien tolérés chez l'Homme (minimisation du risque de réaction allergique par immunisation inter-espèce) et possèdent une demi-vie prolongée chez l'Homme, étant donné que la région constante de la chaîne lourde et la totalité de la chaîne légère de ces anticorps sont d'origine humaine.

La présente invention a également pour objet un médicament comprenant un anticorps humanisé de classe IgA ou un fragment de cet anticorps, tels que définis ci-dessus ; un tel anticorps ou son fragment est notamment utilisé en immunothérapie passive (sérothérapie) pour la prévention et le traitement d'une maladie infectieuse ou du cancer.

La présente invention a également pour objet une composition immunogène ou vaccinale, **caractérisée en ce qu**'elle comprend au moins un anticorps humanisé de classe IgA ou un fragment de cet anticorps, tels que définis ci-dessus, associé à un antigène, de préférence sous la forme d'un complexe antigène-anticorps comprenant un anticorps humanisé de classe IgA ou un fragment de cet anticorps dirigé contre ledit antigène ; une telle composition permet à la fois de cibler l'antigène vers l'épithélium des muqueuses et de le protéger de la protéolyse.

La présente invention a également pour objet une composition pharmaceutique, **caractérisée en ce qu**'elle comprend au moins un anticorps humanisé de classe IgA ou un fragment de cet anticorps, tels que définis ci-dessus, associé par tout moyen approprié à un principe actif ; une telle composition permet à la fois de cibler le principe actif vers l'épithélium des muqueuses et de le protéger de la protéolyse.

Selon un mode de réalisation avantageux des compositions selon l'invention, elles contiennent en outre, au moins un véhicule pharmaceutiquement acceptable et éventuellement des substances porteuses et/ou des adjuvants.

Les véhicules pharmaceutiquement acceptables, les substances porteuses et les adjuvants sont ceux classiquement utilisés.

Les adjuvants sont avantageusement choisis dans le groupe constitué par des émulsions huileuses, de la saponine, des substances minérales, des extraits bactériens, de l'hydroxyde d'alumine et le squalène.

Les substances porteuses sont avantageusement sélectionnées dans le groupe constitué par des liposomes unilamellaires, des liposomes multilamellaires, des micelles de saponine ou des microsphères solides de nature saccharidique ou aurifère.

Les compositions selon l'invention, sont administrées par voie générale (orale, intramusculaire, sous-cutané, intra-péritonéale ou intraveineuse) ou par voie locale (oculaire, nasale, vaginale, rectale) ; la dose et le rythme d'administration varient en fonction de l'espèce (humaine ou animale) et de la maladie à traiter.

La présente invention a également pour objet un réactif de diagnostic comprenant un anticorps humanisé de classe IgA ou un fragment de cet anticorps, tels que définis ci-dessus.

La présente invention a également pour objet l'utilisation d'un anticorps humanisé de classe IgA ou un fragment de cet anticorps, tels que définis ci-dessus, pour la préparation d'un médicament destiné à la prévention et au traitement des maladies infectieuses et du cancer.

La présente invention a également pour objet l'utilisation d'un anticorps humanisé de classe IgA ou un fragment de cet anticorps, tels que définis ci-dessus, pour la préparation d'un réactif destiné au diagnostic des maladies infectieuses et du cancer.

Outre les dispositions qui précèdent, l'invention comprend encore d'autres dispositions qui ressortiront de la description qui va suivre, qui se réfère à des exemples d'obtention et d'utilisation des mammifères non-humains transgéniques selon la présente invention ainsi qu'aux dessins annexés dans lesquels :
- la figure 1 illustre la structure du locus IgH modifié obtenu par recombinaison homologue entre le locus IgH murin et le vecteur de ciblage dénommé p-alphalKI, comprenant un fragment de 5,5 kb du gène alpha 1 humain incluant trois exons codant pour les domaines constants CH1, CH2 et CH3 et l'exon de membrane (mb) et une cassette néo bordée de sites LoxP (fragment de 1,6kb), flanqués en amont d'un fragment d'environ 5 kb correspondant à la région JH-Eµ (fragment DQ 52/JH) et en aval d'un autre fragment d'environ 5 kb correspondant au gène Cµ (fragment Cµ).

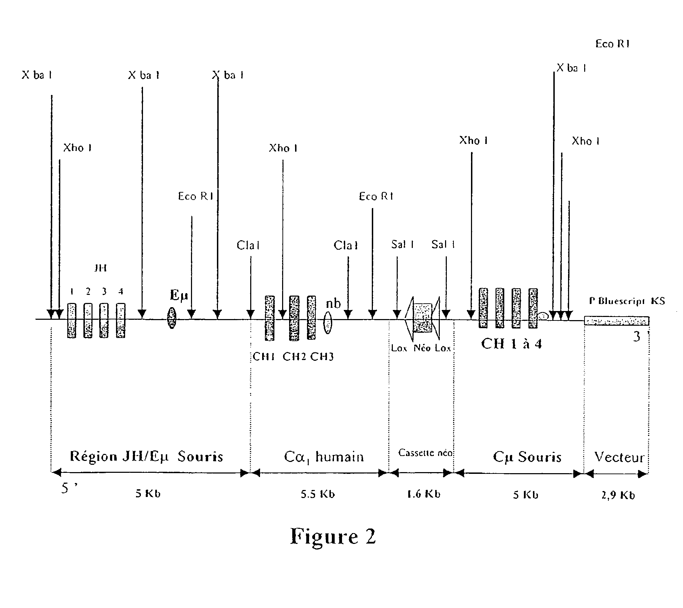

- la figure 2 illustre la structure détaillée du vecteur de ciblage dénommé p-alphalKI, comprenant : un fragment de 5,5 kb du gène alpha 1 humain incluant trois exons codant pour les domaines constants CH1, CH2 et CH3 et l'exon de membrane (mb) et une cassette néo bordée de sites LoxP (fragment de 1,6kb), flanqués en amont d'un fragment d'environ 5 kb correspondant à la région JH-Eµ (fragment DQ 52/JH) et en aval d'un autre fragment d'environ 5 kb correspondant au gène Cµ (fragment Cµ).

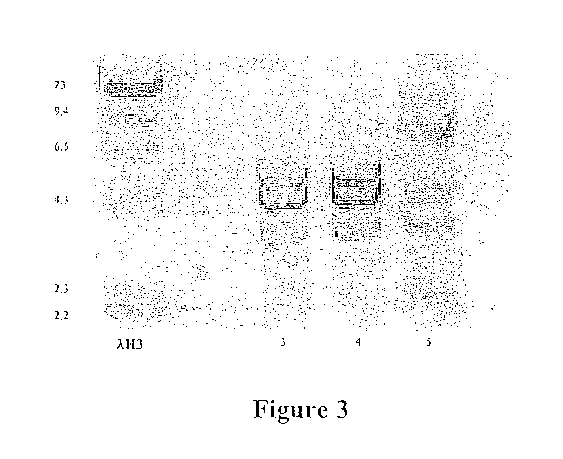

- la figure 3 illustre la confirmation de la séquence du vecteur de ciblage p-alphalKI par restriction enzymatique avec Xho I. λH3 : marqueur de poids moléculaire. Pistes 3 et 4 : clones comprenant la cassette néo insérée dans la bonne orientation ; 5 fragments dont 2 co-migrent (5 kb et 5,3 kb) sont détectés : 6,4 kb (fragment CH2 + CH3 d'α1-cassette néo), 5 kb (fragment Cµ), 5,3 kb (fragment JH + fragment CH1 d'α1) et 3,7 kb (fragment plasmidique + fragment 5' DQ52). Piste 5 : clone comprenant la cassette néo insérée en orientation inverse ; 4 fragments sont détectés : 9,5 kb (fragment JH -fragment CH2 + CH3 d'α1-cassette néo), 5 kb (fragment Cµ), 3,7 kb (fragment plasmidique+ fragment 5' DQ52) et 2,4 kb (fragment CH1 d'α1 + cassette néo).

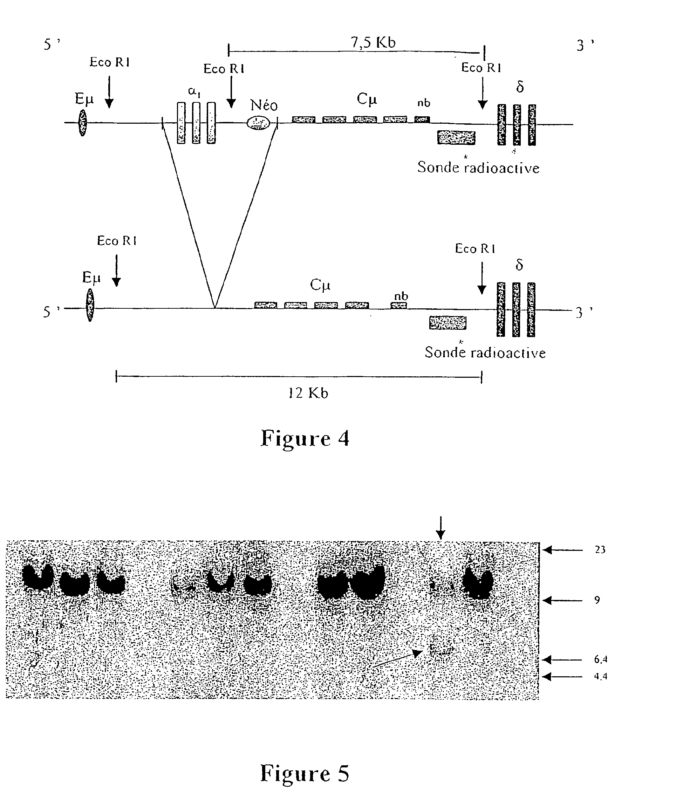

- la figure 4 illustre le profil en Southern-blot d'un allèle recombinant, par comparaison avec un allèle sauvage ; l'ADN génomique digéré par Eco RI est hybridé avec une sonde située en 5' du gène δ.
- la figure 5 illustre l'analyse par Southern-blot de l'ADN génomique des clones ES transfectés avec le vecteur de ciblage p-alphalKI ; l'ADN génomique digéré par Eco RI est hybridé avec une sonde correspondant à la région 5' du gène δ. La flèche indique un clone ayant intégré le transgène α1 humain par recombinaison homologue (fragment de 7,5 kb correspondant à l'allèle recombinant et fragment de 12 kb correspondant à l'allèle sauvage).

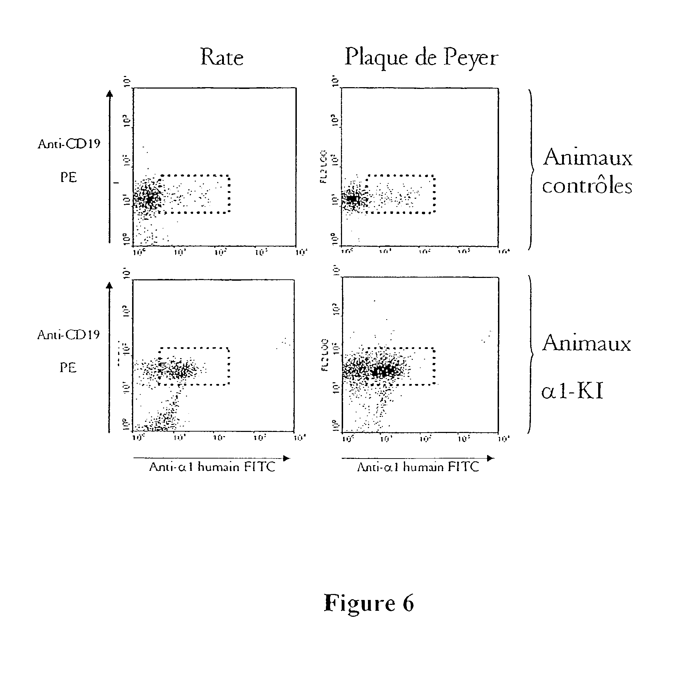

- la figure 6 illustre l'analyse par cytométrie de flux de l'expression d'un récepteur membranaire de la classe des IgA humaines à la surface des lymphocytes périphériques des animaux homozygotes de la lignée transgénique alphalKI. L'axe des abscisses représente le marquage avec un anticorps anti-α1 humain marqué à la fluorescéine et l'axe des ordonnées représente le marquage avec un anticorps anti-CD 19 murin marqué à la phyco-érythrine. Le rectangle en pointillé indique les cellules exprimant à la fois CD19 (cellules B) et une chaîne lourde α1 humaine.

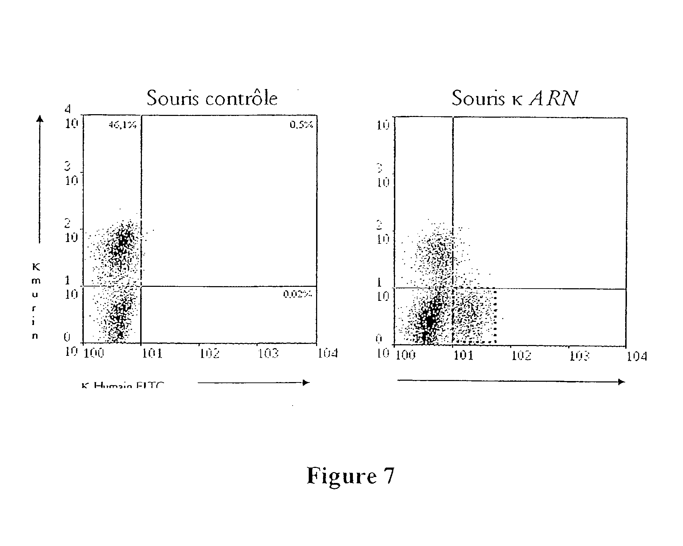

- la figure 7 illustre l'analyse en cytométrie de flux, de l'expression de la chaîne légère kappa humaine à la surface des lymphocytes B périphériques des souris de la lignée kappa ARN, par comparaison avec des souris non-transgéniques (contrôle). L'axe des abscisses représente le marquage avec un anticorps anti-kappa humain marqué à la fluorescéine et l'axe des ordonnées représente le marquage avec un anticorps anti-kappa murin marqué à la phycoérythrine.

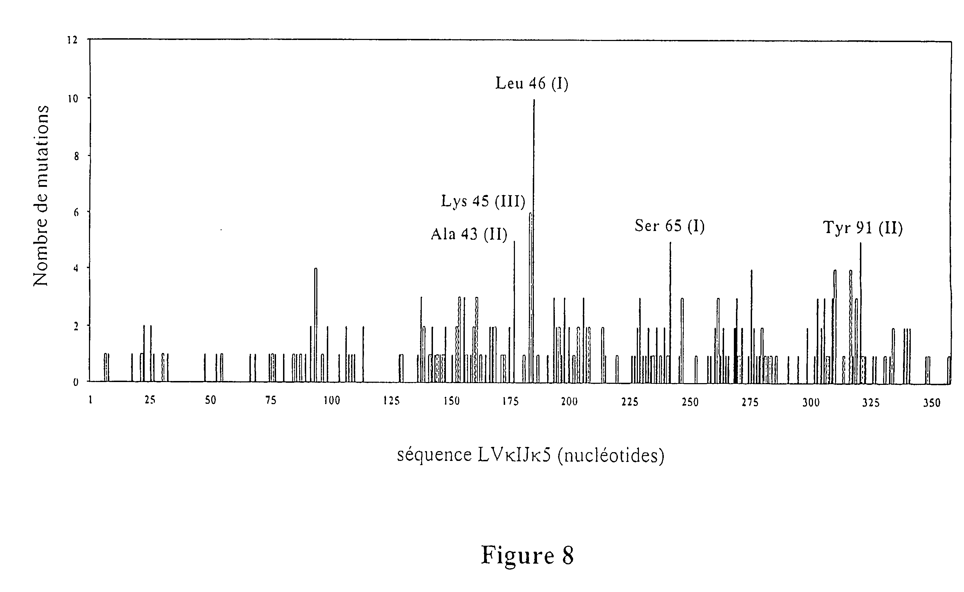

- la figure 8 illustre l'hypermutation somatique du transgène kappa humain dans la lignée de souris transgéniques κ ARN ; la distribution des mutations le long de la chaîne légère kappa humaine de 40 clones isolées à partir de cellules B activés par la PNA a été analysée. Les mutations générant une substitution d'acides aminés, les mutations silencieuses et les mutations générant un codon stop sont indiquées respectivement par ■, □, et ▨. Les acides aminés correspondant aux points d'hypermutation sont indiqués par leur nature et leur position, ainsi que par la position de la mutation dans le codon (en chiffre romain, entre parenthèse).

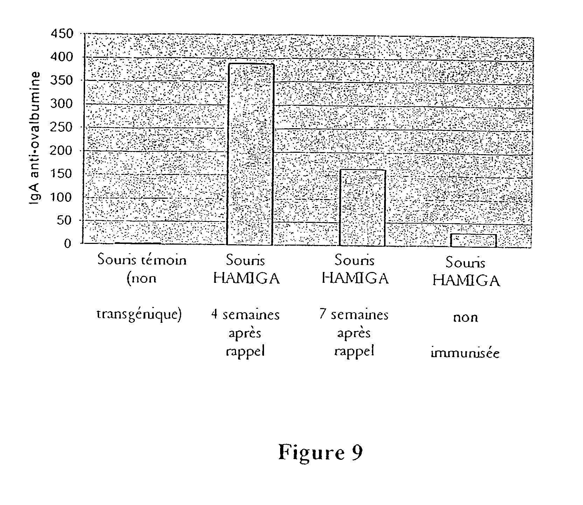

- la figure 9 illustre l'analyse en ELISA de la réponse en anticorps IgA1 chimériques humaines spécifiques, chez les souris doubles-transgéniques de la lignée HAMIGA immunisées avec l'antigène ovalbumine. Les résultats sont exprimés en unités arbitraires d'IgA anti-ovalbumine.

### Exemple 1 : Obtention et caractérisation de la lignée transgénique alpha1KI (alpha1 Knock-In) exprimant une chaîne lourde alpha 1 d'immunoglobuline humaine, chimérique

Le gène alpha 1 humain, incluant les trois exons codant pour les domaines constants CH1, CH2 et CH3 et l'exon de membrane (mb), a été inséré par recombinaison homologue, en lieu et place de la région de commutation Sµ de la chaîne lourde murine (Sµ), de façon à bloquer la commutation de classe vers les gènes constants d'immunoglobulines situés en aval de Cµ sur le locus endogène (locus IgH murin, figure 1). La région ciblée abolit l'expression du gène µ endogène responsable de la synthèse de chaînes lourdes d'IgM, et diminue fortement celle des autres gènes de chaînes lourdes d'immunoglobulines. En conséquence, la lignée transgénique obtenue produit une forte quantités d'IgA chimériques dont le domaine constant humanisé correspond à l'isotype IgA1.

### 1) Construction du vecteur de ciblage de la recombinaison homologue

Les constructions plasmidiques ont été réalisées à partir du plasmide bluescript SK (pSK) (STRATAGENE) et de la souche bactérienne *E. coli* TG1(STRATAGENE), en utilisant les protocoles classiques de préparation, de clonage et d'analyse de l'ADN tels que ceux décrits dans Current Protocols in Molecular Biology (Frederick M. AUSUBEL, 2000, Wiley and son Inc, Library of Congress, USA).

Le vecteur de recombinaison homologue ou vecteur de ciblage dérivé de pSK, dénommé p-alpha1KI (figure 2), comprend : un fragment de 5,5 kb du gène alpha 1 humain incluant trois exons codant pour les domaines constants CH1, CH2 et CH3 et l'exon de membrane (mb) et une cassette néo (fragment de 1,6kb), flanqué en amont d'un fragment d'environ 5 kb correspondant à la région JH-Eµ (fragment DQ 52/JH) et en aval d'un autre fragment d'environ 5 kb correspondant au gène Cµ (fragment Cµ).

De manière plus précise, les différents fragments ont été insérés dans le plasmide bluescript SK, selon les étapes suivantes :
Dans une première étape, le fragment Cµ correspondant aux positions 140101 à 145032 du chromosome 12 murin (Genbank/EMBL AC073553) a été amplifié par PCR à l'aide d'amorces spécifiques appropriées puis cloné au site *Xho I* de pSK pour donner le plasmide pA.
Dans une seconde étape, le fragment DQ 52/JH correspondant aux positions 131281 à 136441 du chromosome 12 murin (Genbank/EMBL AC073553) a été amplifié par PCR à l'aide d'amorces spécifiques appropriées puis cloné en 5' du fragment Cµ, entre les sites *EcoR V* et *Cla I* du plasmide pA, pour donner le plasmide pB.
Dans une troisième étape, la cassette néo décrite dans Pinaud et al., Immunity, 2001, 15, 187-199, a été insérée au site *Sal I* entre DQ52/JH et Cµ, pour donner le plasmide pC.

Le fragment Sac I-Bam HI de 5,5 kb d'un plasmide recombinant comprenant la totalité du gène alpha 1 humain, incluant les séquences des exons CH 1, CH2 et CH3 (Genbank/EMBL J00220) et de l'exon de membrane (Genbank/EMBL X64133) a été ligaturé à chacune de ses extrémités avec des adaptateurs Cla I.

Enfin, dans une dernière étape, le fragment de 5,5 kb flanqué d'adaptateurs Cla I ainsi obtenu a été inséré entre le fragment JH et la cassette néo au site *Cla I* du plasmide pC pour donner le vecteur de ciblage dénommé p-alpha1KI.

La séquence de p-alpha1KI a été vérifiée par séquençage automatique et par analyse de restriction avec les enzymes *Cla I* et *Xho I* (figure 3).

### 2) Transfection de cellules ES et injection dans des blastocystes

Les clones de cellules ES issues de la lignée 129/SJ ont été isolés, analysés puis injectés dans des blastocystes de souris C57/Black 6, en utilisant les protocoles classiques de transgénèse et d'analyse de l'ADN génomique, tels que ceux décrits dans et dans Current Protocols in Molecular Biology (Frederick M. AUSUBEL, 2000, Wiley and son Inc, Library of Congress, USA).

De manière plus précise, des cellules ES ont été transfectées par électroporation de l'ADN de p-alpha1KI linéarisé au site *Not I.* Les clones sélectionnés en présence de généticine ont été prélevés et l'ADN génomique digéré par *EcoR I* a été analysé par Southern Blot à l'aide d'une sonde radioactive s'hybridant en dehors du site de recombinaison homologue, en 5' du gène constant Delta (δ) et de son site *EcoR I* (figure 4) ; cette sonde amplifiée par PCR à l'aide d'amorces spécifiques appropriées, correspond aux positions 140101 à 145032 de la séquence du chromosome 12 murin (EMBL/Genbank AC073553).

La présence d'un allèle recombinant est visualisée par un fragment d'environ 7,5 kb (représentant le fragment µ murin et la cassette néo) alors que l'allèle sauvage correspond à un fragment de 12 kb (figure 5). Dans ces conditions, sur 303 clones analysés, 4 se sont révélés positifs.

La vérification du caryotype de deux des quatre clones recombinants n'a montré aucune anomalie chromosomique (aneuploïdie).

Ces clones ont été injectés dans des blastocystes de souris C57/Black 6 en utilisant les protocoles classiques de transgénèse tels que ceux décrits dans *Transgenic Mouse: Methods and Protocols*, précité. Parmi les souris obtenues, celles présentant le degré de chimérisme le plus élevé ont été analysées par PCR et par ELISA. Une lignée de souris homozygotes pour le locus IgH recombiné, dénommée ci-après lignée alpha 1 *knock-in* ou *alpha1KI*, a ensuite été obtenue par croisement des animaux hétérozygotes présentant le degré de chimérisme le plus élevé.

### 3) Détection du locus IgH recombiné portant le gène Cα1 humain (allèle alpha 1 knock-in ou alpha1KI) et du locus IgH sauvage (allèle µ sauvage) par PCR

L'ADN génomique d'un échantillon de queue des animaux homozygotes obtenus comme précisé ci-dessus, a été analysé par PCR à l'aide des deux couples d'amorces suivants :
- couple spécifique du locus IgH murin non muté (allèle µ sauvage) :
   - ***amorce UpstreamSpe** I* ***Smu* :** 5' GAG TAC CGT TGT CTG GGT CAC 3' (SEQ ID NO:1)
   - ***amorce SacI-3'Imu* :** 5' GAG CTC TAT GAT TAT TGG TTA AC 3' (SEQ ID NO:2)

   La réaction d'amplification a été réalisée avec une température d'hybridation de 61°C. Cette PCR amplifie en 30 cycles un fragment de 91 paires de bases-encadrant le site Spe I-spécifique du locus IgH murin non muté.
- couple spécifique du locus IgH recombiné portant le gène Cα1 humain (allèle alpha 1 *knock-in* ou *alpha1KI*) :
   - amorce NeoI : 5' GCA TGA TCT GGA CGA AGA GCA T 3' (SEQ ID NO:3)
   - amorce Neo2 : 5' TCC CCT CAG AAG AAC TCG TCA A 3' (SEQ ID NO:4)

La réaction d'amplification a été réalisée avec une température d'hybridation de 55°C. Cette PCR amplifie en 30 cycles un fragment de 120 paires de bases spécifique du locus IgH recombiné portant le gène Cα1 humain (mutation alpha *1 knock-in* ou *alpha1KI*).

Une lignée de souris homozygotes pour la mutation alpha1KI, dénommée ci-après lignée alpha 1 *knock-in* ou *alpha1KI* a été établie ; les animaux de cette lignée sont systématiquement et simultanément négatifs en PCR avec les amorces spécifiques de l'allèle µ sauvage et positifs en PCR avec les amorce spécifiques de l'allèle alpha 1 *knock-in*.

### 4) Dosage des IgA sériques totales en néphélométrie et en ELISA

### a) néphélométrie

Les IgA sériques ont été dosées par néphélométrie sur un automate BNII™ (BEHRING) en utilisant le kit de dosage des IgA (BEHRING), selon les recommandations du fournisseur.

Le dosage des IgA sériques a donné des résultats totalement corrélés avec ceux du génotypage réalisé par PCR :
- les animaux contrôle non mutants ont un taux nul d'immunoglobulines humaines de classe IgA
- les animaux hétérozygotes α1-KI ont également un taux indétectable d'IgA humaines et un taux normal d'IgM murines.
- les animaux homozygotes α1-KI ont un taux significatif d'IgA humaines, ce taux variant entre 0,4 et 0,6 g/l dans le sérum. Par contre, les IgM murines sont indétectables dans le sérum de ces animaux.

### b) ELISA

Les résultats obtenus en néphélométrie ont été confirmés en ELISA suivant les étapes suivantes : des plaques à 96 puits (Maxisorb™, NUNC) ont été recouvertes, soit d'anticorps non marqué anti-IgA humaines, soit d'anticorps non-marqué anti-IgM murines, par incubation une nuit à + 4° C en présence de Fab'2 de chèvre anti-IgA humaines ou anti-IgM murines (Southern Biotechnologies Associates), dilués au 1/500^{ème} en tampon carbonate 0,1 M, pH 8,3 (100 microlitres/puits). Après 3 lavages avec du tampon PBS contenant 0,1 % de Tween (PBS-Tween 0,1 %), les plaques ont été saturées en présence de PBS contenant 10 % de sérum de veau foetal (100 microlitres/puits). Après 3 lavages avec du tampon PBS-Tween 0,1%, les sérums à tester, dilués au 1/100^{ème} et au 1/500^{ème} en tampon PBS contenant 10% sérum de veau foetal ont été ajoutés (100 microlitres/puits) et les plaques ont été incubées 3 heures à 37°C. Après 3 lavages avec du tampon PBS-Tween 0,1 %, un antisérum anti-IgA humaines marqué à la phosphatase alcaline ou un sérum anti-IgM murines marqué à la phosphatase alcaline (Biosys) dilués au 1/1000^{ème} dans du PBS-Tween 0,1% (100 microlitres/puits) ont été ajoutés et les plaques ont été incubées 1 heure à 37°C. Après 3 lavages avec du tampon PBS-Tween 0,1%, les IgA et les IgM fixées ont été révélées par addition de substrat de la phosphatase alcaline (p-nitrophenylphosphate, SIGMA) à 1 mg/ml en tampon Tris 0,2M, pH 7,0. La réaction a été bloquée par addition de soude 0,5N (50 microlitres/puits) puis l'absorbtion a été mesurée à une longueur d'onde de 405 nm.

Les données quantitatives sont obtenues par extrapolation avec une gamme d'un sérum étalon (BEHRING) pour le dosage des IgA humaines, et d'une IgM monoclonale murine (SOUTHERN BIOTECHNOLOGIES ASSOCIATES) pour le dosage des IgM murines.

Le dosage des IgA sériques en ELISA montre une différence significative entre les homozygotes et les hétérozygotes ; les sérums d'homozygotes contiennent 0,4 et 0,6 g/l d'IgA1, alors que des valeurs d'absorbance nulles ou très faibles sont observées pour les sérums d'hétérozygotes même à la dilution la plus faible (au 1/100^{ème}). A l'inverse lorsque les IgM murines sont dosées en ELISA, on observe un taux normal d'IgM murines (de l'ordre de 1 g/l) chez les souris contrôles « non mutantes » de même que chez les animaux hétérozygotes pour la mutation α1-KI. Par contre, le taux des IgM murines est nul chez les animaux homozygotes pour la mutation α1-KI.

### 5) Recherche de l'expression d'un récepteur membranaire de la classe des IgA humaines à la surface des lymphocytes périphériques des animaux mutants

Les animaux homozygotes porteurs de la mutation α1-KI ont été phénotypés en cytométrie de flux, par double marquage à l'aide d'anticorps spécifiques d'IgA1 humaine ou d'IgM murines marqués à la fluorescéine, et d'anticorps spécifiques des cellules B (anticorps anti-CD19) marqués à la phycoérythrine. De manière plus précise :
- Préparation des cellules lymphoïdes : deux organes lymphoïdes périphériques : la rate et les plaques de Peyer, ont été prélevés séparément chez les animaux mutants homozygotes α1KI, dilacérés en tampon versène (Invitrogen), et filtrés sur tamis (40 microns) afin d'obtenir une suspension de cellules individuelles débarrassée des agrégats cellulaires. Les cellules spléniques ont alors été centrifugées et soumises à une étape supplémentaire de choc osmotique afin de lyser les globules rouges par remise en suspension du culot cellulaire dans 1 ml d'eau distillée. Les cellules des échantillons ont ensuite été immédiatement remises en suspension dans du milieu complet (RPMI + 10% sérum de veau foetal), dénombrées et conservées sur de la glace.
- Marquage à l'aide d'anticorps fluorescents : 10⁵ cellules de chaque échantillon ont été incubées pendant 30 minutes à 4° C avec une dilution au 1/100^{ème}, soit d'un anticorps anti-IgM de souris marqué à l'isothiocyanante de fluorescéine (Southem Biotechnologies), soit d'un anticorps anti-IgA humaines marqué à l'isothiocyanante de fluorescéine, ou bien avec la combinaison d'un des anticorps précédents avec un anticorps spécifique des cellules B (anticorps anti-CD 19) marqué à la phycoérythrine (double marquage). Les cellules ont ensuite été lavées dans 5 ml de PBS puis le surnageant a été décanté et les cellules ont été remises en suspension dans 100 microlitres de PBS, 0,5% BSA, 0,1 mM EDTA.
- Analyse en cytofluorimétrie : les cellules marquées ont été analysées à l'aide d'un cytomètre de flux (COULTER XL™).

Les résultats de la cytométrie de flux sont en accord avec ceux du dosage des immunoglobulines sériques. Chez les animaux homozygotes de la lignée alpha1-KI, aucune expression d'IgM murines n'est détectée, ni dans la rate, ni dans les plaques de Peyer.

Pourtant en l'absence d'expression d'IgM, un compartiment de cellules B périphériques CD19+ est capable de se différencier chez ces animaux et représente 10 à 12 % des lymphocytes spléniques ou 40 à 60% des lymphocytes des plaques de Peyer. Ce compartiment exprime des IgA membranaires dont la chaîne lourde humanisée est reconnue par un anticorps spécifique des IgA1 et marqué à la fluorescéine (figure 6).

### Exemple 2 : Obtention et caractérisation de la lignée transgénique κ ARN exprimant une chaîne légère kappa d'immunoglobuline humaine

Une lignée d'animaux transgéniques exprimant dans toutes leurs cellules B, une chaîne légère kappa humaine codée par la région variable VκI-Jκ5 et la région Cκ (chaîne kappa ARN, EMBL/Genbank X64133), a été obtenue par transgénèse directe, à partir du vecteur d'expression décrit dans Chauveau et al, Gene, 1998, 222, 279-285.

### 1) Construction du vecteur de transgénèse

Le vecteur de transgénèse est le plasmide pALIEµ décrit dans Chauveau et al, Gene, 1998, 222, 279-285 ; il contient à la fois le promoteur VH et l'enhancer Eµ en 5' de la cassette codant pour la chaîne kappa ARN, et en 3' de cette cassette : les trois enhancer hs3a, hs12 et hs3b, situés en 3' du locus IgH en 3'. La séquence codante correspond à la chaîne VκI-Jκ5-Cκ (Genbank/EMBL X64133). Le plasmide pALIEµ a été linéarisé par les enzymes de restriction *Not I* et *Pvu I* qui coupent à l'intérieur de la séquence plasmidique, *Not I* étant situé en amont du promoteur qui précède le segment Vκ cloné et *Pvu I* étant situé au sein du gène de résistance à l'ampicilline porté par le plasmide. Le fragment incluant l'ensemble de la cassette d'expression kappa flanquée de tous les éléments promoteurs et régulateurs de l'expression a ensuite été inséré de façon aléatoire dans des blastocystes de souris en utilisant les protocoles classiques de transgénèse directe tels que ceux décrits dans *Transgenic Mouse: Methods and Protocols*, précité.

### 2) Identification des animaux fondateurs de la lignée κ ARN et typage de leur descendance

Une lignée de souris transgénique possédant le transgène κ ARN a été obtenue après injection du vecteur d'expression ; la présence de ce transgène humain a été vérifiée sur l'ADN des souris par Southern blot à l'aide d'une sonde spécifique de la région Cκ humaine (fragment *EcoRI-EcoRI* de 2,5 kb incluant la totalité de l'exon Cκ humain). Les animaux portant l'insertion du transgène sur les deux allèles du point d'insertion (animaux homozygotes) ont une quantité double du transgène et peuvent être distingués par le Southern blot des animaux portant une seule copie du transgène (animaux hétérozygotes). Alternativement, la présence du transgène a été détectée par PCR à l'aide d'amorces permettant d'amplifier spécifiquement la séquence humaine VκI-Jκ5-Cκ (Genbank/EMBL X64133).

### 3) Recherche de l'expression des chaînes légères kappa humaines à la surface des lymphocytes périphériques des souris de la lignée kappa ARN

Les animaux dizygotes porteurs du transgène kappa ARN ont été phénotypés en cytométrie de flux, par double marquage à l'aide d'un anticorps anti-K murin (marqué à la phycoérythrine) en conjonction avec un anticorps anti-κ humain (marqué à l'isothiocyanante de fluorescéine), selon le protocole tel que décrit à l'exemple 1.

Ces animaux montrent une expression du transgène κ humain sur la majorité des cellules B (figure 7). De plus le transgène induit un phénomène d'exclusion allélique tel que les cellules B exprimant le transgène κ humain n'expriment pas de gène endogène de chaînes légères de souris. En cytométrie, ces cellules sont donc positives lors du marquage avec l'antisérum anti-chaînes κ humaines et négatives avec l'antisérum anti chaînes κ murines (figure 7).

### 4) Analyse de l'hypermutation somatique du transgène κ chez les souris de la lignée kappa ARN

Il a également été montré que cette chaîne légère κ humaine est capable de s'associer avec des chaînes lourdes et de se diversifier grâce au phénomène d'hypermutation somatique (déclenché par une réponse à l'antigène). Ce transgène respectant l'architecture endogène d'un gène κ avec présence de l'intron Jκ-Cκ entre le VκJκ et le Cκ, bénéficie en outre d'une expression forte assurée par la combinaison promoteur P_{VH} / enhancer Eµ + palindrome régulateur 3'IgH (hs3a, hs1,2, hs3b). L'action cumulative de tous ces éléments régulateurs permet de recruter la machinerie d'hypermutation somatique au niveau du transgène. De manière plus précise, les plaques de Peyer des souris transgéniques sont prélevées par dissection de l'intestin. La suspension cellulaire est préparée en broyant les plaques de Peyer à travers une membrane de nylon. Les cellules sont lavées trois fois à + 4° C, dans du DMEM contenant 10 % de sérum de veau foetal. Les cellules mortes ont été éliminées après chaque lavage et la suspension cellulaire a été ajustée à 10⁶ cellules/ml.

Les cellules ont été incubées 30 min à + 4° C en présence d'anticorps anti-B220 biotinylé. Après deux lavages avec du DMEM contenant 5 % de sérum de veau foetal, les cellules ont été incubées 30 min à + 4° C en présence de streptavidine couplée à la phycoérythrine, puis lavées et remises en suspension dans du PBS contenant 5 % de sérum de veau foetal. Après addition d'une lectine spécifique des cellules B activées (PNA pour *peanut agglutinin*) conjuguée à la FITC, la a suspension cellulaire a été incubée 30 min à + 4° C. Après deux lavages avec du DMEM, les cellules ont été remises en suspension dans du DMEM puis elles B ont ensuite été triées, par cytométrie en flux, en deux populations : B220⁺PNA^{high} (B activées) et B220⁺PNA^{low}(B au repos).

L'ADN génomique a été extrait des deux populations cellulaires triées à l'aide du kit QIAamp Tissue (QIAGEN). Une amplification par réaction de polymérisation en chaîne (PCR) a été réalisée sur 2µl d'ADN génomique en utilisant des amorces correspondant à la région signal du Vκ1 humain (5'-AAGTCGACATGGACATGAGGGTGCC-3') (SEQ ID NO:5) et au début de la région Jκ5 humaine (5'-TTCTCGAGACTTAGGTTTAATCTCCAG-3') (SEQ ID NO:6). Le programme d'amplification consistait en : une étape initiale de dénaturation à 94°C pendant 5 min; suivi de 35 cycles alternant une étape de dénaturation à 94°C pendant 30 s, une étape d'hybridation à 52°C pendant 30 s, et une étape d'élongation à 72°C pendant 30 s; puis une étape finale d'élongation à 72°C pendant 7 min.

Le produit d'amplification a été purifié sur gel d'agarose à 1, 2%, élué (kit QIAquick Gel Extraction, QIAGEN) puis cloné dans le vecteur pCRII-TOPO (INVITROGEN). Les clones recombinants ont été testés par restriction enzymatique puis purifiés (kit Flexiprep, PHARMACIA) et séquencés par la méthode de Sanger. Les réactions de séquençage ont été réalisées par PCR à l'aide des amorces M13 reverse et M13(-20) et de didésoxynucléotides fluorescents puis analysées par électrophorèse capillaire sur séquenceur automatique (ABI-PRISM 310, PERKIN-ELMER). Les séquences obtenues à partir des cellules B activées ont ensuite été alignées avec la séquence originale du transgène non muté (Genbank/EMBL X64133). Le nombre et la position des mutations ont été analysés (figure 8). Le transgène κ subit cette hypermutation somatique à un taux pratiquement aussi élévé (17 mutations pour 1000 bases) que les gènes d'immunoglobulines endogènes (qui mutent à un taux de 40 mutations pour 1000 bases). Ce transgène unique est donc capable de générer un « répertoire » kappa possédant une certaine diversité.

### Exemple 3 : Obtention et caractérisation de la lignée double-transgénique HAMIGA exprimant une chaîne lourde alpha 1 chimérique et une chaîne légère kappa d'immunoglobulines humaines.

Le croisement des lignées κARN et alpha1-KI décrites aux exemples précédents, génère des souris doubles transgéniques κARN / alpha1-KI.

Pour ce faire, les animaux homozygotes pour la mutation alpha1-KI et homozygotes pour le transgène κ ARN ont été croisés entre eux. En première génération (F1) après ce croisement, tous les animaux obtenus sont hétérozygotes pour la mutation alpha1-KI et hétérozygotes pour le transgène κ ARN. Ces animaux F1 ont donc été à nouveau croisés : à la génération suivante (F2) les lois de la génétique Mendélienne permettent d'obtenir 1 animal sur 4 homozygote pour la mutation alpha1-KI et un animal sur 4 homozygote pour le transgène κ ARN. Parmi ces animaux F2, un animal sur 16 a donc pu être sélectionné comme portant à la fois la mutation alpha1-KI à l'état homozygote et comme portant le transgène κ ARN à l'état homozygote. Ces animaux sont les fondateurs de la lignée HAMIGA et ils transmettent de façon stable à leur descendance les gènes permettant simultanément la production d'une chaîne lourde alpha1 humanisée en remplacement de la production d'IgM murines ainsi que la production et la diversification par hypermutation d'une chaîne κ humaine.

Cette lignée de souris doubles transgéniques est dénommé lignée HAMIGA pour « *Humanized Antibodies Made Up Of Monoclonal Immunoglobulin A* ».

### 1) Obtention de la lignée double-transgénique HAMIGA

### a) présence du transgène κ

La transmission du transgène κ ARN au cours des croisement d'animaux transgéniques a été suivie par Southern Blot à l'aide d'une sonde spécifique de la région Cκ humaine (fragment e *EcoRI-EcoRI* de 2,5 kb incluant la totalité de l'exon Cκ humain).

L'expression du transgène κ chez les animaux mutants a été détectée par dosage en ELISA de chaînes kappa libres humaines éliminées dans les urines des animaux. De manière plus, précise : des plaques à 96 puits (Maxisorb®, NUNC) ont été incubées une nuit à + 4° C en présence d' un anticorps non marqué anti-K humain (Kallestad) dilué au 1/1000^{ème} en tampon carbonate 0,1 M pH 8,3 (100 microlitres/puits). Après 3 lavages avec du tampon PBS contenant 0,1 % de Tween (PBS-Tween 0,1%), les plaques ont été saturées en présence de PBS contenant 10% sérum de veau foetal (100 microlitres/puits). Après 3 lavages avec du tampon PBS-Tween 0,1%, les échantillons d'urine à tester, dilués au 1/100^{ème} et au 1/500^{ème} en tampon PBS contenant 10% de sérum de veau foetal ont été ajoutés (100 microlitres/puits) et les plaques ont été incubées 3 heures à 37°C. Après 3 lavages avec du tampon PBS-Tween 0,1%, un antisérum anti-κ humain marqué à la phosphatase alcaline (SIGMA) dilué au 1/1000^{ème} dans du PBS-Tween 0,1% (100 microlitres/puits) a été ajouté et les plaques ont été incubées 1 heure à 37°C. Après 3 lavages avec du tampon PBS-Tween 0,1%, les chaînes légères kappa humaines fixées ont été révélées par addition de substrat de la phosphatase alcaline (p-nitrophényl-phosphate, SIGMA) à 1 mg/ml en tampon Tris 0,2M, pH 7,0. La réaction a été bloquée par addition de soude 0,5N (50 microlitres/puits) puis l'absorbtion a été mesurée à une longueur d'onde de 405 nm.

Alternativement, l'expression du transgène kappa humain a été analysée par cytométrie de flux comme décrit à l'exmple 2. Les résultats montrent que la présence du transgène κ ARN entraîne un phénomène important d'exclusion allélique, tel que parmi les lymphocytes périphériques plus de 50 % expriment la chaîne légère humaine et ne réarrangent donc pas les gènes de chaîne légères murines pour exprimer une chaîne légère murine.

### b ) homozygotie pour la mutation α1-KI

Le premier élément simple indiquant l'homozygotie α1-KI est la présence d'un taux élevé d'IgA1 humaines dans le sérum des animaux. En outre l'homozygotie a été confirmée en PCR par la positivité de la « PCR α1-KI » associée à la négativité de la « PCR allèle µ sauvage ». Enfin, après sacrifice des animaux, l'analyse en cytométrie de flux a permis de montrer sur les lymphocytes de la rate et des plaques de Peyer que la totalité des lymphocytes B (CD19+) expriment des IgA1 humaines membranaire alors qu'en parallèle aucune cellule B n'exprime d'IgM murine.

### c) Vérification de la présence simultanée de la mutation alpha1-KI et du transgène κ ARN chez les animaux HAMIGA et leur descendance.

Les animaux double-transgéniques HAMIGA ont été caractérisés comme ceux répondant simultanément aux deux spécificités décrites ci-dessus : présence du transgène κ ARN à l'état homozygote et homozygotie pour la mutation alpha1-KI. De plus, ces animaux se reproduisent en préservant ces deux spécificités et le phénotype de leur descendance possède les propriétés suivantes, simultanément et de façon stable :
- la production d'IgA1 humanisée à un taux conséquent (facilement vérifiable par ELISA ou néphélométrie sur un simple prélèvement de sang réalisé chez les animaux vivants au niveau du sinus rétro-orbitaire)
- la production de chaîne légère κ humaine (facilement vérifiable par ELISA sur un simple prélèvement d'urines réalisé chez les animaux vivants).

### 2) Immunisation des animaux

Les animaux ont été immunisés une première fois par injection intra-péritonéale de 10 microgrammes d'ovalbumine (SIGMA) diluée dans 100 microlitres de sérum physiologique et mise en émulsion avec 200 microlitres d'adjuvant complet de Freund (SIGMA).

Après 4 semaines, les animaux ont subi un rappel vaccinal par injection intra-péritonéale de 10 microgrammes d'ovalbumine (SIGMA) diluée dans 100 microlitres de sérum physiologique et mise en émulsion avec 200 microlitres d'adjuvant incomplet de Freund (SIGMA).

### 3) Dosage des anticorps spécifiques de l'antigène vaccinal (ovalbumine)

La présence d'anticorps spécifiques de l'antigène vaccinal ovalbumine a été analysée en ELISA, 4 semaines, puis 7 semaines après la deuxième injection de l'antigène, en suivant la technique suivante : des plaques à 96 puits (Maxisorb®, NUNC) ont été incubées une nuit à + 4° C en présence d'ovalbumine à la concentration de 10 microgrammes/ml en tampon carbonate 0,1 M pH 8,3 (100 microlitres/puits). Après 3 lavages avec du tampon PBS contenant 0,1 % de Tween (PBS-Tween 0,1%), les plaques ont été saturées en présence de PBS contenant 10% de sérum de veau foetal (100 microlitres/puits). Après 3 lavages avec du tampon PBS-Tween 0,1%, les échantillons de sérum à tester, dilués au 1/20^{ème} et au 1/100^{ème} en tampon PBS contenant 10% de sérum de veau foetal ont été ajoutés (100 microlitres/puits) et les plaques ont été incubées 3 heures à 37°C. Après 3 lavages avec du tampon PBS-Tween 0,1%, un antisérum anti-IgA humaines marqué à la phosphatase alcaline (BIOSYS) dilué au 1/1000^{ème} dans du PBS-Tween 0,1% (100 microlitres/puits) a été ajouté et les plaques ont été incubées 1 heure à 37°C. Après 3 lavages avec du tampon PBS-Tween 0,1%, les chaînes légères kappa humaines fixées ont été révélées par addition de substrat de la phosphatase alcaline (p-nitrophenylphosphate, SIGMA) à 1 mg/ml en tampon Tris 0,2M, pH 7,0. La réaction a été bloquée par addition de soude 0,5N (50 microlitres/puits) puis l'absorbtion a été mesurée à une longueur d'onde de 405 nm. Le taux d'anticorps IgA anti-ovalbumine a été exprimé en unités arbitraires établies pour les sérums dilués au 1/100^{ème} en fonction du rapport Densité optique sérum testé / Densité oprtique du sérum témoin.

Les résultats présentés à la figure 9 montrent la présence d'anticorps spécifiques de l'antigène vaccinal ovalbumine 4 semaines (taux des anticorps IgAl humaines anti-ovalbumine à 388 unités), puis 7 semaines après la deuxième injection de l'antigène (taux des anticorps IgA anti-ovalbumine à 162 unités). En parallèle, il a également été verifié qu'en l'absence d'immunisation des animaux, le taux des anticorps IgA anti-ovalbumine détectés restait inférieur à 30 unités.

Le répertoire de réponse aux antigènes de ces souris est attendu comme subnormal puisqu'on sait que c'est essentiellement le domaine VH de la chaîne lourde qui contribue à la formation du site anticorps (or la chaîne lourde transgénique α1 humaine bénéficie d'un répertoire totalement diversifié puisque correspondant au répertoire normal généré par les réarrangements des segments VH, D et JH du locus IgH murin.). Ces souris sont capables de produire des anticorps de forte affinité en réponse secondaire, ce qui résulte du fait que leurs lymphocytes B peuvent recruter le phénomène d'hypermutation somatique à la fois au niveau du gène de chaîne lourde et du transgène de chaîne légère κ *ARN*.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

## Revendications

1. Mammifère non-humain transgénique, **caractérisé en ce qu'**il comprend un locus IgH modifié par remplacement de la séquence de commutation Sµ par tout ou partie d'un transgène constitué par le gène Cα d'une immunoglobuline humaine de classe A incluant au moins l'exon codant pour le domaine CH3 et l'exon de membrane.

2. Mammifère non-humain transgénique selon la revendication 1, **caractérisé en ce qu'**il est homozygote pour ledit locus IgH modifié.

3. Mammifère non-humain transgénique selon la revendication 1 ou la revendication 2, **caractérisé en ce que** ledit locus IgH est modifié par remplacement de la séquence de commutation Sµ, par la totalité du gène Cα.

4. Mammifère non-humain transgénique selon la revendication 1 ou la revendication 2, **caractérisé en ce que** ledit locus IgH est modifié par remplacement de la séquence de commutation Sµ, par le segment du gène Cα incluant l'exon codant pour le domaine CH3 et l'exon de membrane.

5. Mammifère non-humain transgénique selon l'une quelconque des revendications 1 à 4, **caractérisé en ce que** ledit gène Cα est Cα1.

6. Mammifère non-humain transgénique selon l'une quelconque des revendications 1 à 5, **caractérisé en ce qu'**il comprend un autre transgène codant pour une chaîne légère d'immunoglobuline humaine.

7. Mammifère non-humain transgénique selon la revendication 6, **caractérisé en ce que** la dite chaîne légère est une chaîne kappa.

8. Mammifère non-humain transgénique selon la revendication 7, **caractérisé en ce que** ledit transgène comprend l'activateur intronique Eµ en amont et le palindrome *hs3*α/*hs1,2*/*hs3b* en aval.

9. Mammifère non-humain transgénique selon la revendication 8, **caractérisé en ce que** ledit transgène est sous le contrôle du promoteur de la chaîne lourde d'immunoglobulines humaine.

10. Mammifère non-humain transgénique selon l'une quelconque des revendications 6 à 9, **caractérisé en ce qu'**il est dizygote pour ledit transgène.

11. Mammifère non-humain transgénique selon l'une quelconque des revendications 6 à 10, **caractérisé en ce qu'**il possède un locus endogène de la chaîne kappa inactivé.

12. Mammifère non-humain transgénique selon la revendication 11, **caractérisé en ce qu'**il est homozygote pour ledit locus endogène de la chaîne kappa inactivé.

13. Mammifère non-humain transgénique selon l'une quelconque des revendications 1 à 12, **caractérisé en ce qu'**il possède un gène codant pour la chaîne J inactivé.

14. Mammifère non-humain transgénique selon la revendication 13, **caractérisé en ce qu'**il est homozygote pour ledit gène codant pour la chaîne J inactivé.

15. Mammifère non-humain transgénique selon la revendication 13 ou la revendication 14, **caractérisé en ce qu'**il comprend un autre transgène codant pour une chaîne J d'immunoglobuline humaine.

16. Mammifère non-humain transgénique selon une quelconque des revendications 1 à 15, **caractérisé en ce qu'**il s'agit d'une souris transgénique.

17. Souris transgénique selon la revendication 16, **caractérisée en ce qu'**elle comprend :
- un locus IgH modifié par remplacement de la séquence de commutation Sµ par tout le gène Cα1 d'une immunoglobuline humaine de classe A, et
- un gène Vκ complet comprenant le gène VκI réarrangé avec un gène Jκ5, l'intron Jκ-Cκ et le gène Cκ, sous le contrôle transcriptionnel du promoteur de la chaîne lourde humaine (pVH) et de l'activateur intronique Eµ en amont, et du palindrome *hs3*α/*hs1,2*/*hs3b* en aval.

18. Vecteur de ciblage de recombinaison homologue, **caractérisé en ce qu'**il comprend le gène Cα d'une immunoglobuline humaine de classe A ou un segment de ce gène incluant au moins l'exon codant pour le domaine CH3 et l'exon de membrane, flanqué des fragments de séquences du locus IgH d'un mammifère non-humain qui sont adjacents à la séquence Sµ.

19. Vecteur de ciblage selon la revendication 18, **caractérisé en ce qu'**il comprend une cassette d'expression d'un marqueur de sélection approprié, adjacent audit gène Cα ou à un segment dudit gène.

20. Vecteur de ciblage selon la revendication 19, **caractérisé en ce que** ladite cassette d'expression est flanquée de séquences de recombinaison site-spécifique.

21. Vecteur de ciblage selon la revendication 19, **caractérisé en ce que** lesdites séquences sont les séquences LoxP de la recombinase Cre.

22. Vecteur de ciblage selon l'une quelconque des revendications 18 à 21, **caractérisé en ce que** lesdits fragments de séquences qui sont adjacents à la séquence Sµ sont d'origine murine.

23. Vecteur de ciblage selon la revendication 22, **caractérisé en ce que** le gène Cα ou le segment dudit gène est flanqué, respectivement en 5' et en 3', des fragments correspondants aux positions 131281 à 136441 et 140101 à 145032 dans la séquence du chromosome 12 murin (numéro d'accès AC073553 dans la base de données EMBL/Genbank).

24. Cellule embryonnaire de mammifère non-humain, modifiée par un vecteur de ciblage selon l'une quelconque des revendications 18 à 23.

25. Utilisation d'un mammifère non-humain transgénique selon l'une quelconque des revendications 1 à 16 ou d'une souris transgénique selon la revendication 17, pour la production d'anticorps humanisés de classe IgA ou de fragments de ces anticorps.

26. Procédé de préparation d'anticorps humanisés de classe IgA ou de fragments de ces anticorps, **caractérisé en ce qu'**il comprend au moins les étapes suivantes :
- l'immunisation d'un mammifère non-humain transgénique selon l'une quelconque des revendications 1 à 16 ou d'une souris transgénique selon la revendication 17, et
- l'obtention par tout moyen approprié, d'anticorps humanisés de classe IgA ou de fragments de ces anticorps, à partir du sérum, des sécrétions ou des lymphocytes B dudit mammifère non-humain transgénique préalablement sacrifié.

27. Anticorps humanisé de classe IgA susceptible d'être obtenu par le procédé selon la revendication 26, **caractérisé en ce qu'**il comprend une chaîne lourde chimérique dont les domaines constants sont d'origine humaine et une chaîne légère humaine dont le domaine variable est codé par VκI-Jκ5.

28. Fragment d'un anticorps humanisé de classe IgA selon la revendication 27, **caractérisé en ce qu'**il comprend un fragment desdites chaînes lourdes et légères.

29. Fragment d'anticorps humanisé de classe IgA selon la revendication 28, **caractérisé en ce qu'**il est sélectionné dans le groupe constitué par les fragments Fab, Fab'2 et Fc.

30. Médicament, **caractérisé en ce qu'**il comprend un anticorps humanisé de classe IgA selon la revendication 27 ou un fragment de cet anticorps selon la revendication 28 ou la revendication 29.

31. Réactif de diagnostic, **caractérisé en ce qu'**il comprend un anticorps humanisé de classe IgA selon la revendication 27 ou un fragment de cet anticorps selon la revendication 28 ou la revendication 29.

32. Composition immunogène ou vaccinale, **caractérisée en ce qu'**elle comprend au moins un anticorps humanisé de classe IgA selon la revendication 27 ou un fragment de cet anticorps selon la revendication 28 ou la revendication 29, associé à un antigène.

33. Composition pharmaceutique, **caractérisée en ce qu'**elle comprend au moins un anticorps humanisé de classe IgA selon la revendication 27 ou un fragment de cet anticorps selon la revendication 28 ou la revendication 29, associé par tout moyen approprié à un principe actif.

34. Utilisation d'un anticorps humanisé de classe IgA selon la revendication 27 ou d'un fragment de cet anticorps selon la revendication 28 ou la revendication 29, pour la préparation d'un médicament destiné à la prévention et au traitement des maladies infectieuses et du cancer.

35. Utilisation d'un anticorps humanisé de classe IgA selon la revendication 27 ou d'un fragment de cet anticorps selon la revendication 28 ou la revendication 29, pour la préparation d'un réactif destiné au diagnostic des maladies infectieuses et du cancer.

## Claims

1. A non-human transgenic mammal, **characterized in that** it comprises an IgH locus modified by replacement of the switch sequence Sµ by all or part of a transgene consisting of the Cα gene of a class A human immunoglobulin, including at least the exon coding for the CH3 domain and the membrane exon.

2. The non-human transgenic mammal according to claim 1, **characterized in that** it is homozygous for the said modified IgH locus.

3. The non-human transgenic mammal according to claim 1 or claim 2, **characterized in that** the said IgH locus is modified by replacement of the switch sequence Sµ by the entire Cα gene.

4. The non-human transgenic mammal according to claim 1 or claim 2, **characterized in that** the said IgH locus is modified by replacement of the switch sequence Sµ by the segment of the Cα gene including the exon coding for the CH3 domain and the membrane exon.

5. The non-human transgenic mammal according to any one of claims 1 to 4, **characterized in that** the said Cα gene is Cα1.

6. The non-human transgenic mammal according to any one of claims 1 to 5, **characterized in that** it comprises another transgene coding for a human immunoglobulin light chain.

7. The non-human transgenic mammal according to claim 6, **characterized in that** the said light chain is a kappa chain.

8. The non-human transgenic mammal according to claim 7, **characterized in that** the said transgene comprises the intronic activator Eµ upstream and the palindrome *hs3a*/*hs1,2*/*hs3b* downstream.

9. The non-human transgenic mammal according to claim 8, **characterized in that** the said transgene is under the control of the promoter of the human immunoglobulin heavy chain.

10. The non-human transgenic mammal according to any one of claims 6 to 9, **characterized in that** it is dizygous for the said transgene.

11. The non-human transgenic mammal according to any one of claims 6 to 10, **characterized in that** it has an inactivated endogenous locus of the kappa chain.

12. The non-human transgenic mammal according to claim 11, **characterized in that** it is homozygous for the said inactivated endogenous locus of the kappa chain.

13. The non-human transgenic mammal according to any one of claims 1 to 12, **characterized in that** it has an inactivated gene coding for the J chain.

14. The non-human transgenic mammal according to claim 13, **characterized in that** it is homozygous for the said inactivated gene coding for the J chain.

15. The non-human transgenic mammal according to claim 13 or claim 14, **characterized in that** it comprises another transgene coding for a human immunoglobulin J chain.

16. The non-human transgenic mammal according to any one of claims 1 to 15, **characterized in that** it is a transgenic mouse.

17. A transgenic mouse as claimed in claim 16, **characterized in that** it comprises:
- an IgH locus modified by replacement of the switch sequence Sµ by the entire Cα1 gene of a class A human immunoglobulin, and
- a complete Vκ gene comprising the VκI gene rearranged with a Jκ5 gene, the Jκ-Cκ intron and the Cκ gene, under the transcriptional control of the promoter of the human heavy chain (pVH) and of intronic activator Eαµ upstream and of the palindrome *hs3a*/*hs1,2*/*hs3b* downstream.

18. A homologous recombination targeting vector, **characterized in that** it comprises the Cα gene of a class A human immunoglobulin or a segment of this gene including at least the exon coding for the CH3 domain and the membrane exon, flanked by fragments of sequences of the IgH locus of a non-human mammal which are adjacent to the Sµ sequence.

19. The targeting vector according to claim 18, **characterized in that** it comprises an expression cassette of a suitable selection marker adjacent to the said Cα gene or to a segment of the said gene.

20. The targeting vector according to claim 19, **characterized in that** the said expression cassette is flanked by site-specific recombination sequences.

21. The targeting vector according to claim 19, **characterized in that** the said sequences are LoxP sequences of the Cre recombinase.

22. The targeting vector according to any one of claims 18 to 21, **characterized in that** the said fragments of sequences which are adjacent to the Sµ sequence are of murine origin.

23. The targeting vector according to claim 22, **characterized in that** the Cα gene or the segment of the said gene is flanked at 5' and at 3' respectively, by fragments corresponding to positions 131281 to 136441 and 140101 to 145032 in the sequence of murine chromosome 12 (accession number AC073553 in the EMBL/Genbank database).

24. A embryonic cell of a non-human mammal, modified by a targeting vector according to any one of claims 18 to 23.

25. The use of a non-human transgenic mammal according to any one of claims 1 to 16 or of a transgenic mouse according to claim 17 for the production of humanized class IgA antibodies or of fragments of these antibodies.

26. A process for the preparation of humanized class IgA antibodies or of fragments of these antibodies, **characterized in that** it comprises at least the following steps:
- the immunization of a non-human transgenic mammal according to any one of claims 1 to 16 or of a transgenic mouse according to claim 17, and
- the obtaining by any suitable means of humanized class IgA antibodies or of fragments of these antibodies from serum, secretions or B lymphocytes of the said non-human transgenic mammal sacrificed beforehand.

27. A humanized class IgA antibody obtainable by the process according to claim 26, **characterized in that** it comprises a chimaeric heavy chain, the constant domains of which are of human origin, and a human light chain, the variable domain of which is coded by VκI-Jκ5.

28. A humanized class IgA antibody fragment according to claim 27, **characterized in that** it comprises a fragment of the said heavy and light chains.

29. The humanized class IgA antibody fragment according to claim 28, **characterized in that** it is selected from the group consisting of the fragments Fab, Fab'2 and Fc.

30. A medicament, **characterized in that** it comprises a humanized class IgA antibody according to claim 27 or a fragment of this antibody according to claim 28 or claim 29.

31. A diagnostic reagent, **characterized in that** it comprises a humanized class IgA antibody according to claim 27 or a fragment of this antibody according to claim 28 or claim 29.

32. An immunogenic or vaccine composition, **characterized in that** it comprises at least one humanized class IgA antibody according to claim 27 or a fragment of this antibody according to claim 28 or claim 29, associated with an antigen.

33. A pharmaceutical composition, **characterized in that** it comprises at least one humanized class IgA antibody according to claim 27 or a fragment of this antibody according to claim 28 or claim 29, associated with an active principle by any suitable means.

34. Use of a humanized class IgA antibody according to claim 27 or of a fragment of this antibody according to claim 28 or claim 29 for the preparation of a medicament for prevention and treatment of infectious diseases and cancer.

35. Use of a humanized class IgA antibody according to claim 27 or of a fragment of this antibody according to claim 28 or claim 29 for the preparation of a reagent for diagnosis of infectious diseases and cancer.

## Patentansprüche

1. Nicht-menschlisches transgenes Säugetier, **dadurch gekennzeichnet, dass** es einen IgH-Locus umfasst, der durch Ersatz der Kommutationssequenz Sµ durch ein Transgen, als ganzes oder einen Teil davon, das durch das Gen Cα eines menschlichen Immunglobulins der Klasse A, umfassend wenigstens das Exon, das für die Domäne CH3 codiert, und das Membranexon, gebildet wird, modifiziert ist.

2. Nicht-menschliches transgenes Säugetier gemäß Anspruch 1, **dadurch gekennzeichnet, dass** es bezüglich des modifizierten IgH-Locus homozygot ist.

3. Nicht-menschliches transgenes Säugetier gemäß Anspruch 1 oder Anspruch 2, **dadurch gekennzeichnet, dass** der IgH-Locus durch Ersatz der Kommutationssequenz Sµ durch die Gesamtheit des Gens Cα modifiziert ist.

4. Nicht-menschliches transgenes Säugetier gemäß Anspruch 1 oder Anspruch 2, **dadurch gekennzeichnet, dass** der IgH-Locus durch Ersatz der Kommutationssequenz Sµ durch das Segment des Gens Cα, das das Exon, das für die Domäne CH3 codiert, und das Membranexon umfasst, modifiziert ist.

5. Nicht-menschliches transgenes Säugetier gemäß einem der Ansprüche 1 bis 4, **dadurch gekennzeichnet, dass** das Gen Cα Cα1 ist.

6. Nicht-menschliches transgenes Säugetier gemäß einem der Ansprüche 1 bis 5, **dadurch gekennzeichnet, dass** es ein anderes Transgen umfasst, das für eine leichte Kette von menschlichem Immunglobulin codiert.

7. Nicht-menschliches transgenes Säugetier gemäß Anspruch 6, **dadurch gekennzeichnet, dass** die leichte Kette eine Kappa-Kette ist.

8. Nicht-menschliches transgenes Säugetier gemäß Anspruch 7, **dadurch gekennzeichnet, dass** das Transgen den intronischen Aktivator Eµ stromaufwärts und das Palindrom *hs3a*/*hs1,2*/*hs3b* stromabwärts umfasst.

9. Nicht-menschliches transgenes Säugetier gemäß Anspruch 8, **dadurch gekennzeichnet, dass** das Transgen unter der Kontrolle des Promotors der schweren Kette von menschlichem Immunglobulin ist.

10. Nicht-menschliches transgenes Säugetier gemäß einem der Ansprüche 6 bis 8, **dadurch gekennzeichnet, dass** es bezüglich des genannten Transgens dizygot ist.

11. Nicht-menschliches transgenes Säugetier gemäß einem der Ansprüche 6 bis 10, **dadurch gekennzeichnet, dass** es einen inaktivierten endogenen Locus der Kappa-Kette besitzt.

12. Nicht-menschliches transgenes Säugetier gemäß Anspruch 11, **dadurch gekennzeichnet, dass** es bezüglich des inaktivierten endogenen Locus der Kappa-Kette homozygot ist.

13. Nicht-menschliches transgenes Säugetier gemäß einem der Ansprüche 1 bis 12, **dadurch gekennzeichnet, dass** es ein inaktiviertes Gen besitzt, das für die Kette J codiert.

14. Nicht-menschliches transgenes Säugetier gemäß Anspruch 13, **dadurch gekennzeichnet, dass** es bezüglich des Gens, das für die inaktivierte Kette J codiert, homozygot ist.

15. Nicht-menschliches transgenes Säugetier gemäß Anspruch 13 oder Anspruch 14, **dadurch gekennzeichnet, dass** es ein anderes Transgen umfasst, das für eine Kette J von menschlichem Immunglobulin codiert.

16. Nicht-menschliches transgenes Säugetier gemäß einem der Ansprüche 1 bis 15, **dadurch gekennzeichnet, dass** es sich um eine transgene Maus handelt.

17. Transgene Maus gemäß Anspruch 16, **dadurch gekennzeichnet, dass** sie umfasst:
- einen IgH-Locus, der durch Ersatz der Kommutationssequenz Sµ durch das ganze Gen Cα1 eines menschlichen Immunglobulins der Klasse A modifiziert ist, und
- ein vollständiges Gen Vκ, das das Gen VκI, neu geordnet mit einem Gen Jκ5, das Intron Jκ-Cκ und das Gen Cκ umfasst, unter der Transkriptionskontrolle des Promotors der menschlichen schweren Kette (pVH) und des intronischen Aktivators Eµ stromaufwärts und des Palindroms *hs3a*/*hs1,2*/*hs3b* stromabwärts.

18. Targeting-Vektor für die homologe Rekombination, **dadurch gekennzeichnet, dass** er das Gen Cα eines menschlichen Immunglobulins der Klasse A oder ein Segment dieses Gens, das wenigstens das Exon, das für die Domäne CH3 codiert, und das Membranexon umfasst, flankiert von Fragmenten der Sequenzen des IgH-Locus eines nicht-menschlichen Säugetiers, die an die Sequenz Sµ angrenzen, umfasst.

19. Targeting-Vektor gemäß Anspruch 18, **dadurch gekennzeichnet, dass** er eine Expressionskassette eines geeigneten Selektionsmarkers, benachbart zu dem Gen Cα oder einem Segment des genannten Gens, umfasst.

20. Targeting-Vektor gemäß Anspruch 19, **dadurch gekennzeichnet, dass** die Expressionskassette von Sequenzen der ortsspezifischen Rekombination flankiert wird.

21. Targeting-Vektor gemäß Anspruch 19, **dadurch gekennzeichnet, dass** die Sequenzen die Sequenzen LoxP der Rekombinase Cre sind.

22. Targeting-Vektor gemäß einem der Ansprüche 18 bis 21, **dadurch gekennzeichnet, dass** die Fragmente von Sequenzen, die an die Sequenz Sµ angrenzen, muriner Herkunft sind.

23. Targeting-Vektor gemäß Anspruch 22, **dadurch gekennzeichnet, dass** das Gen Cα oder das Segment des Gens in 5' bzw. in 3' von Fragmenten flankiert wird, die den Positionen 131281 bis 136441 und 140101 bis 145032 in der Sequenz des murinen Chromosoms 12 (Eingangsnummer AC073553 in der Datenbank EMBL/Genbank) entsprechen.

24. Embryonale nicht-menschliche Säugerzelle, modifiziert durch einen Targeting-Vektor gemäß einem der Ansprüche 18 bis 23.

25. Verwendung eines nicht-menschlichen transgenen Säugetiers gemäß einem der Ansprüche 1 bis 16 oder einer transgenen Maus gemäß Anspruch 17 für die Produktion von humanisierten Antikörpern der Klasse IgA oder von Fragmenten dieser Antikörper.

26. Verfahren zur Herstellung von humanisierten Antikörpern der Klasse IgA oder von Fragmenten dieser Antikörper, **dadurch gekennzeichnet, dass** es wenigstens die folgenden Stufen umfasst:
- Immunisierung eines nicht-menschlichen transgenen Säugetiers gemäß einem der Ansprüche 1 bis 16 oder einer transgenen Maus gemäß Anspruch 17 und
- Erhalt, durch jedes geeignete Mittel, von humanisierten Antikörpern der Klasse IgA oder von Fragmenten dieser Antikörper aus Serum, Ausscheidungen oder Lymphozyten B des nicht-menschlichen transgenen Säugetiers, das vorher getötet wurde.

27. Humanisierter Antikörper der Klasse IgA, der durch das Verfahren gemäß Anspruch 26 erhalten werden kann, **dadurch gekennzeichnet, dass** er eine chimäre schwere Kette, deren konstante Domänen menschlicher Herkunft sind, und eine menschliche leichte Kette, deren variable Domäne durch VκI-Jκ5 codiert wird, umfasst.

28. Fragment eines humanisierten Antikörpers der Klasse IgA gemäß Anspruch 27, **dadurch gekennzeichnet, dass** es ein Fragment der genannten schweren und leichten Ketten umfasst.

29. Fragment eines humanisierten Antikörpers der Klasse IgA gemäß Anspruch 28, **dadurch gekennzeichnet, dass** es aus der Gruppe selektioniert ist, die aus den Fragmenten Fab, Fab'2 und Fc besteht.

30. Medikament, **dadurch gekennzeichnet, dass** es einen humanisierten Antikörper der Klasse IgA gemäß Anspruch 27 oder ein Fragment dieses Antikörpers gemäß Anspruch 28 oder Anspruch 29 umfasst.

31. Diagnosereagenz, **dadurch gekennzeichnet, dass** es einen humanisierten Antikörper der Klasse IgA gemäß Anspruch 27 oder ein Fragment dieses Antikörpers gemäß Anspruch 28 oder Anspruch 29 umfasst.

32. Immunogene oder vakzinale Zusammensetzung, **dadurch gekennzeichnet, dass** sie wenigstens einen humanisierten Antikörper der Klasse IgA gemäß Anspruch 27 oder ein Fragment dieses Antikörpers gemäß Anspruch 28 oder Anspruch 29, assoziiert mit einem Antigen, umfasst.

33. Pharmazeutische Zusammensetzung, **dadurch gekennzeichnet, dass** sie wenigstens einen humanisierten Antikörper der Klasse IgA gemäß Anspruch 27 oder ein Fragment dieses Antikörpers gemäß Anspruch 28 oder Anspruch 29, assoziiert durch jedes geeignete Mittel mit einem Wirkstoff, umfasst.

34. Verwendung eines humanisierten Antikörpers der Klasse IgA gemäß Anspruch 27 oder eines Fragments dieses Antikörpers gemäß Anspruch 28 oder Anspruch 29 für die Herstellung eines Medikaments, das zur Prävention oder Behandlung von infektiösen Krankheiten und Krebs bestimmt ist.

35. Verwendung eines humanisierten Antikörpers der Klasse IgA gemäß Anspruch 27 oder eines Fragments dieses Antikörpers gemäß Anspruch 28 oder Anspruch 29 für die Herstellung eines Reagenzes, das für die Diagnose von infektiösen Krankheiten und Krebs bestimmt ist.
